(19) Europäisches Patentamt
European Patent Office
Office européen des brevets

(11) EP 0 842 148 B1

(12) FASCICULE DE BREVET EUROPEEN

(45) Date de publication et mention de la délivrance du brevet: **20.12.2000 Bulletin 2000/51**

(21) Numéro de dépôt: **96927119.6**

(22) Date de dépôt: **01.08.1996**

(51) Int Cl.[7]: **C07C 311/37**, A61K 31/18

(86) Numéro de dépôt international: **PCT/FR96/01215**

(87) Numéro de publication internationale: **WO 97/06136 (20.02.1997 Gazette 1997/09)**

(54) **DERIVES DE BENZENESULFONAMIDE, LEUR PREPARATION ET LEURS APPLICATIONS EN THERAPEUTIQUE**

BENZOLSULFONAMIDDERIVATE, IHRE HERSTELLUNG UND IHRE THERAPEUTISCHE VERWENDUNG

BENZENESULPHONAMIDE DERIVATIVES, PREPARATION THEREOF AND THERAPEUTICAL USES THEREOF

(84) Etats contractants désignés:
**AT BE CH DE DK ES FI FR GB GR IE IT LI LU NL PT SE**

(30) Priorité: **04.08.1995 FR 9509503**

(43) Date de publication de la demande: **20.05.1998 Bulletin 1998/21**

(73) Titulaire: **Sanofi-Synthélabo**
**75013 Paris (FR)**

(72) Inventeurs:
- **PURCELL, Thomas**
  **F-78490 Montfort-L'Amaury (FR)**
- **PHILIPPO, Christophe**
  **F-92500 Rueil-Malmaison (FR)**

(74) Mandataire: **Thouret-Lemaitre, Elisabeth et al**
**Sanofi-Synthélabo**
**Service Brevets**
**174, avenue de France**
**75013 Paris (FR)**

(56) Documents cités:
**EP-A- 0 034 432**
**EP-A- 0 202 164**
**FR-A- 2 405 931**
**US-A- 3 860 647**
**US-A- 3 943 254**

- **CHEMICAL AND PHARMACEUTICAL BULLETIN, vol. 30, no. 11, Novembre 1982, TOKYO, JP, pages 4092-4101, XP002001065 T. FUJIKURA, ET AL.: "Studies on benzenesulphonamid derivatives with alpha- and beta-adrenergic antagonistic and antihypertensive activites"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 842 148 B1

Printed by Jouve, 75001 PARIS (FR)

## Description

La présente invention a pour objet des dérivés de benzène sulfonamide, leur préparation et leurs applications en thérapeutique.

FR-A-2 405 931 décrit des dérivés de phényléthanolamine de formule (II)

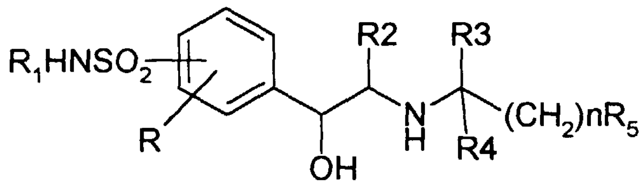

(II)

dans laquelle R5 représente un groupe aryle, un noyau benzodioxane, un groupe aryloxy ou arylthio; qui peuvent être utilisés comme hypotenseurs.

EP 0 034 432 présente des dérivés de phényléthylamine de formule (III)

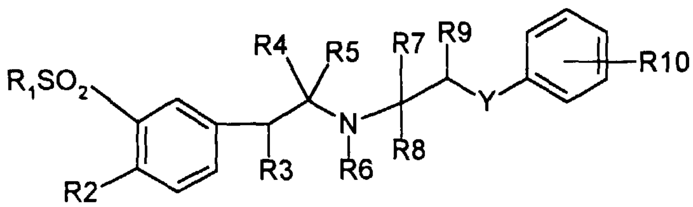

(III)

qui peuvent être utilisés dans le traitement de l'hypertension.

La publication dans Chem. & Pharm. Bulletin, v. 30, n°11, 1982, présente plus spécifiquement les composés de formule (III) dans laquelle R1 représente un amino, R3 représente un hydroxy, R4, R5, R6, R7 et R9 représentent un hydrogène, R8 représente un groupe méthyle et Y représente un méthylène; et démontre leur activité hypotensive.

US 3,860,647 divulgue des composés de formule (IV)

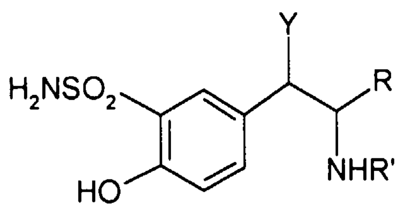

(IV)

et leur activité en tant qu'agents bloquant des récepteurs β-adrénergiques utiles dans le traitement des maladies cardio-vasculaires.

US 3,943,254 est une division du brevet US 3,860,647 et revendique les compositions pharmaceutiques comprenant un composé de formule (IV).

Les composés de l'invention répondent à la formule générale (I)

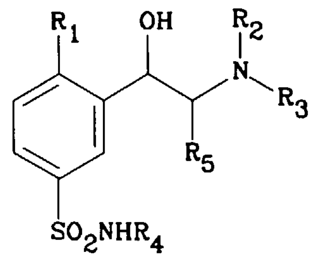

(I)

dans laquelle :

$R_1$ représente un atome d'hydrogène ou d'halogène, tel que chlore ou fluor, ou un groupe $C_{1-4}$ alkyle ou $C_{1-4}$ alcoxy, linéaire ou ramifié,
$R_2$, $R_3$, et $R_4$ représentent, indépendamment les uns des autres, des atomes d'hydrogène ou des groupes $C_{1-4}$ alkyle, linéaire, ramifié ou cyclique, et
$R_5$ représente un atome d'hydrogène, un groupe $C_{1-2}$ alkyle, $C_{1-2}$ fluoroalkyle, ou $C_{1-2}$ perfluoroalkyle.

Le terme $C_{1-4}$ alkyle comprend les radicaux linéaires, à chaîne ramifiée, ou cyclisée ayant jusqu'à 4 atomes de carbone comprenant le méthyle, l'éthyle, le propyle, l'isopropyle, le butyle, l'isobutyle et le tertiobutyle, de préférence, $C_{1-2}$ alkyle, tel que le méthyle et l'éthyle.
Le terme $C_{1-2}$ fluoroalkyle comprend les radicaux linéaires, ayant 1 à 2 atomes de carbone comme définis précédemment, dont au moins l'un des atomes d'hydrogène est substitué par un fluor, étant entendu que tous les atomes d'hydrogène ne sont pas substitués par des atomes de fluor. Le terme $C_{1-2}$ perfluoroalkyle comprend les radicaux linéaires, ayant 1 à 2 atomes de carbone comme définis précédemment, dont tous les atomes d'hydrogène sont substitués par un fluor.
Le terme $C_{1-4}$ alcoxy comprend les radicaux linéaires ou ramifiés, ayant jusqu'à 4 atomes de carbone, attachés par un atome d'oxygène, comprenant le méthoxy, l'éthoxy, le propoxy, l'isopropoxy, le butoxy, l'isobutoxy et le tertiobutoxy, de préférence, $C_{1-2}$ alcoxy, le méthoxy et l'éthoxy.
Les composés de formule générale (I) comportent un ou plusieurs atomes de carbone asymétrique. Ils peuvent donc exister sous forme d'énantiomères ou diastéréomères. Ces énantiomères, diastéréoisomères, ainsi que leurs mélanges, y compris les mélanges racémiques, font partie de l'invention.
Les composés de formule générale (I) peuvent se présenter sous forme de sels d'addition à des acides pharmaceutiquement acceptables, qui font également partie de l'invention. Selon la présente invention, les sels préférés sont les sels de méthanesulfonates, d'oxalates et de fumarates.
Les composés de formule générale (I) dans laquelle $R_5$ représente un groupe $C_{1-2}$ alkyle, $C_{1-2}$ fluoroalkyle, ou $C_{1-2}$ perfluoroalkyle, existent sous forme d'isomères syn ou tans. Ces formes ainsi que leurs mélanges font partie de l'invention.
Les composés préférés sont ceux pour lesquels, $R_5$ représente un atome d'hydrogène, un méthyle ou un éthyle, de préférence un hydrogène ou un méthyle, sous forme d'énantiomères ou de diastéréoisomères, ou de mélanges de ces différentes formes, y compris de mélanges racémiques, ainsi que leurs sels d'addition à des acides pharmaceutiquement acceptables.
D'autres composés préférés sont ceux pour lesquels, $R_1$ représente un atome d'hydrogène, un fluor, un chlore ou un groupe $C_{1-4}$ alcoxy, de préférence méthoxy ou éthoxy, sous forme d'énantiomères ou de diastéréoisomères, ou de mélanges de ces différentes formes, y compris de mélanges racémiques, ainsi que leurs sels d'addition à des acides pharmaceutiquement acceptables.
D'autres composés de choix sont ceux pour lesquels, $R_2$ et $R_3$ représentent indépendamment l'un de l'autre, un atome d'hydrogène, un méthyle, un éthyle ou un isopropyle, de préférence un hydrogène, sous forme d'énantiomères ou de diastéréoisomères, ou de mélange de ces différentes formes, y compris de mélanges racémiques, ainsi que leurs sels d'addition à des acides pharmaceutiquement acceptables.
Parmi ceux-ci, on peut citer les composés pour lesquels :

$R_1$ représente un atome d'hydrogène, un fluor, un chlore ou un groupe $C_{1-4}$ alcoxy, de préférence méthoxy ou éthoxy,
$R_2$ et $R_3$ représentent indépendamment l'un de l'autre, un atome d'hydrogène, un méthyle, un éthyle ou un isopropyle, de préférence un hydrogène,

$R_4$ représente, un atome d'hydrogène ou un groupe $C_{1-4}$ alkyle, linéaire, ramifié ou cyclique, et
$R_5$ représente un atome d'hydrogène, un méthyle ou un éthyle, de préférence un atome d'hydrogène ou un méthyle, sous forme d'énantiomères ou de diastéréoisomères, ou de mélanges de ces différentes formes, y compris de mélanges racémiques, ainsi que leurs sels d'addition à des acides pharmaceutiquement acceptables,

et tout particulièrement, on peut citer

le α-(aminométhyl)-2-méthoxy-5-sulfonamidobenzèneméthanol et ses sels,
le (+)-α-(aminométhyl)-2-méthoxy-5-sulfonamidobenzène méthanol et ses sels,
le (-)-α-(aminométhyl)-2-méthoxy-5-sulfonamidobenzène méthanol et ses sels,
le α-(aminométhyl)-2-chloro-5-sulfonamidobenzèneméthanol et ses sels, et
le α-(aminométhyl)-2-fluoro-5-sulfonamidobenzèneméthanol et ses sels.

Les composés de formule générale (I) dans laquelle $R_1$ représente un groupe alcoxy et $R_2$ et $R_3$ représentent des atomes d'hydrogène, peuvent être préparés selon le procédé représenté en annexe 1, qui consiste à traiter un dérivé de benzaldéhyde de formule (V), dans laquelle $R_1$ est défini comme précédemment, par l'orthoformiate d'éthyle, en présence de chlorure d'ammonium, puis par la chlorhydrine sulfurique, à traiter le dérivé de 5-chlorosulfonylbenzaldéhyde de formule (IV) par une amine de formule $R_4NH_2$ dans laquelle $R_4$ est défini comme dans la formule générale (I), à faire réagir ensuite le dérivé de 5-sulfonamidobenzaldéhyde de formule (III) avec le cyanure de triméthylsilyle (TMSCN), en présence d'iodure de zinc, et enfin à réduire le composé de formule (II) ainsi obtenu, par le borohydrure de lithium, en présence de chlorure de triméthylsilyle (TMSC1).
Les composés de formule générale (I) peuvent également être préparés selon le procédé représenté en annexe 2, à partir d'un dérivé de sulfonamidoacétophénone de formule (XII).
Dans le cas où $R_1$ est défini comme dans la formule générale (I), à l'exception de la valeur alkyle, ce procédé consiste à traiter le dérivé de 5-sulfonamidophénylcétone de formule (XII) par le brome, puis à faire réagir le composé de formule (XI), soit avec le chlorure de lithium pour obtenir le composé de formule (X) qui est ensuite réduit par le borane pour donner le composé de formule (IX) puis traité par l'azoture de sodium pour donner le composé de formule (VIII), soit avec l'azoture de sodium puis le borohydrure de sodium pour obtenir le composé de formule (VIII), soit avec le borohydrure de sodium en présence de carbonate de potassium pour obtenir le composé de formule (VII), et enfin à traiter le composé de formule (VIII) par l'hydrogène en présence d'un catalyseur tel que 1 palladium sur charbon, dans le cas où $R_1$ n'est pas un atome, de chlore, ou par la triphénylphosphine, puis par l'ammoniaque, dans le cas où $R_1$ est un atome de chlore, pour obtenir le composé de formule générale (I) dans laquelle $R_2$ et $R_3$ sont des atomes d'hydrogène, ou à traiter le composé de formule (VII) soit par une amine de formule $R_2(R_3)NH$ dans laquelle $R_2$ représente un atome d'hydrogène ou un groupe $C_{1-4}$ alkyle et $R_3$ un groupe $C_{1-4}$ alkyle, pour obtenir le composé de formule générale (I) dans laquelle $R_2$ et $R_3$ sont définis comme précédemment, soit par une amine de formule $R_2(Bn)NH$ dans laquelle $R_2$ est un groupe $C_{1-4}$ alkyle et Bn un groupe benzyle, pour obtenir un composé de formule (VI), qui est ensuite réduit par l'hydrogène en présence d'un catalyseur, tel que le palladium sur charbon, pour donner le composé de formule générale (I) dans laquelle $R_2$ est un groupe alkyle.
Dans le cas où $R_1$ est un groupe alkyle, ce procédé consiste à traiter le composé de formule (XII) par le dichloroiodate de benzyltriméthylammonium pour obtenir le composé de formule (X) dans laquelle $R_1$ est un groupe alkyle, qui est ensuite traité comme indiqué précédemment, pour obtenir le composé de formule générale (I) dans laquelle $R_2$ et $R_3$ sont des atomes d'hydrogène et $R_1$ un groupe alkyle, par l'intermédiaire des composés de formules (IX) et (VIII) correspondants.
Les composés de formule (XII)

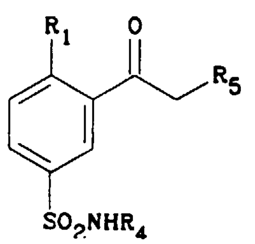

(XII)

dans laquelle $R_1$, $R_4$ et $R_5$ définis comme dans la formule générale (I),peuvent être préparés par réaction d'un dérivé d'phénylcétone de formule (XIV)

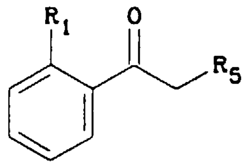

(XIV)

dans laquelle $R_1$ est défini comme dans la formule générale (I), avec la chlorhydrine sulfurique, pour donner un dérivé de chloro-sulfonylphénylcétone de formule (XIII)

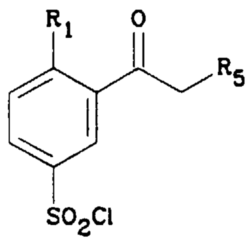

(XIII)

qui est ensuite traité par une amine de formule $R_4NH_2$, dans laquelle $R_4$ est défini comme dans la formule générale (I).

Les composés de formule (XII) dans laquelle $R_4$ représente un atome d'hydrogène peuvent également être préparés par réaction d'un composé de formule (XVII)

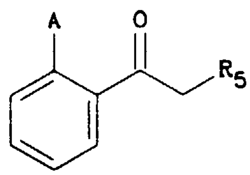

(XVII)

dans laquelle A est identique à $R_1$ tel que défini dans la formule générale (I) ou bien représente un groupe hydroxyle, avec l'acide nitrique, pour obtenir un dérivé de nitro-phénylcétone de formule (XVI)

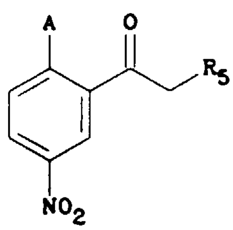

(XVI)

qui est réduit en dérivé d'aminophénylcétone par l'hydrogène en présence de palladium sur charbon ou par le chlorure d'étain, éventuellement après traitement par un iodure d'alkyle, dans le cas où A représente un groupe hydroxyle, pour obtenir le dérivé de 2-alcoxyphénylcétone correspondant, et enfin à traiter le composé de formule (XV)

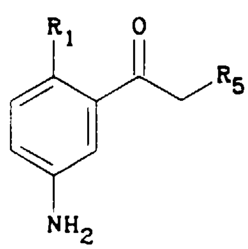

(XV)

par le nitrite de sodium, le chlorure cuivreux et l'anhydride sulfureux.

Les énantiomères des composés de formule générale (I) sont préparés à partir des énantiomères des composés de formule (VIII)

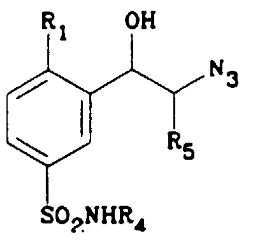

(VIII)

qui sont eux-mêmes obtenus,
soit par synthèse énantiosélective, qui comprend le traitement du composé de formule (XI)

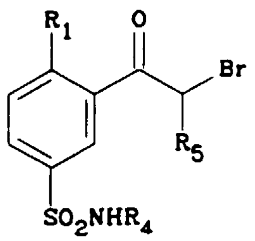

(XI)

par l'azoture de sodium et la réaction du composé de formule (XVIII) ainsi obtenu,

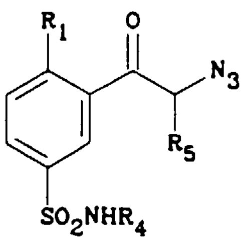

(XVIII)

avec le (+)- ou le (-)-B-chlorodiisopinocampheylborane (DIP-Cl) pour obtenir respectivement les énantiomères (+) et (-) du composé de formule (VIII),
soit par résolution enzymatique du composé (IX)

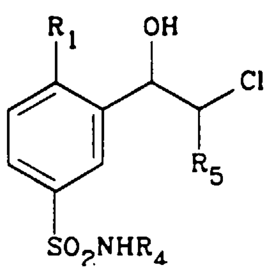

(IX)

qui comprend le traitement du composé de formule (IX) racémique par l'acide acétique, l'hydrolyse enzymatique sélective, par la lipase SP 523 (lipase obtenue par technique d'ADN recombiné à partir d'*Aspergillus orysae*), du composé de formule (XIX) ainsi formé

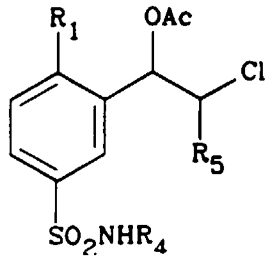

(XIX)

conduisant à l'énantiomère (+) du composé de formule (IX) et à l'énantiomère (-) du composé de formule (XIX) non hydrolysé, la séparation par chromatographie de l'énantiomère (+) du composé de formule (IX) et de l'énantiomère (-) du composé de formule (XIX), et l'hydrolyse de l'énantiomère (-) du composé de formule (XIX) pour obtenir l'énantiomère (-) du composé de formule (IX), et enfin la réaction des énantiomères (+) et (-) du composé de formule (IX) avec l'azoture de sodium,
soit par résolution chimique, qui comprend la réaction du composé de formule (VIII) avec la N-carbobenzyloxy-L-alanine (N-CBZ-alanine), la séparation par chromatographie puis l'hydrolyse des énantiomères du composé de formule (XX)

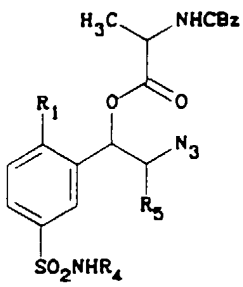

(XX)

Les sels des composés de formule générale (I) sont obtenus par réaction des composés de formule générale (I) sous forme de base avec les acides pharmaceutiquement acceptables.
Les produits de départ sont connus dans la littérature ou directement disponibles dans le commerce.
Les exemples suivants illustrent les procédés et techniques appropriés pour la préparation de cette invention, sans toutefois limiter l'étendue de la revendication. Les microanalyses élémentaires et les spectres RMN et IR confirment les structures des composés obtenus.

Exemple 1 : méthanesulfonate de α-(aminométhyl)-2-méthoxy-5-sulfonamidobenzèneméthanol

1.1. 2-Méthoxy-5-sulfonamidobenzaldéhyde

Ce composé est obtenu selon le procédé décrit dans le brevet FR 73 35277, en faisant passer un courant d'ammoniac dans une solution de 2-méthoxy-5-chlorosulfonylbenzaldéhyde dans du chloroforme.
Selon le même procédé, en traitant le 2-méthoxy-5-chloro sulfonylbenzaldéhyde par 10 équivalents d'amine de formule $R_4NH_2$, pendant 3 heures à température ambiante, on a obtenu les composés suivants :

- le 2-méthoxy-5-méthylsulfonamidobenzaldéhyde.
  Point de fusion : 118°C.
- le 2-méthoxy-5-cyclopropylsulfonamidobenzaldéhyde. Point de fusion : 162°C.
- le 2-méthoxy-5-isopropylsulfonamidobenzaldéhyde.
  Point de fusion : 125°C.
- le 2-méthoxy-5-t-butylsulfonamidobenzaldéhyde.
  Point de fusion : 99°C.

1.2. α-Aminométhyl-2-méthoxy-5-sulfonamidobenzèneméthanol

Dans un ballon de 100 ml, on introduit 10,4 g (48,3 mmol) de 2-méthoxy-5-sulfonamidobenzaldéhyde et 18,4 ml (96,6 mmol) de cyanure de triméthylsilyle, puis on ajoute 0,5 g (1,56 mmol) d'iodure de zinc et on agite le mélange à température ambiante pendant 10 minutes. On ajoute ensuite 20 ml de tétrahydrofuranne anhydre et on transfère la solution dans une ampoule à brome.

D'autre part, dans un ballon de 500 ml, on introduit 100 ml de tétrahydrofuranne anhydre et 2,6 g (119 mmol) de borohydrure de lithium. On agite la solution, puis on ajoute, goutte à goutte, 30 ml (236 mmol) de chlorure de triméthylsilyle et on agite le mélange à température ambiante pendant 10 minutes.
On ajoute alors, goutte à goutte, la solution de triméthyl-silylcyanohydrine préparée précédemment. On agite le mélange pendant 16 heures, puis on ajoute, goutte à goutte, 20 ml d'éthanol et on concentre la solution. On ajoute ensuite, goutte à goutte, 120 ml d'une solution à 20 % d'hydroxyde de potassium et on concentre la solution. On purifie le résidu par chromatographie sur colonne avec un mélange 90/9/1 de dichlorométhane, de méthanol et d'ammoniaque, puis on le recristallise dans l'éthanol et on le sèche au dessicateur sous vide sur anhydride phosphorique.
On obtient 0,30 g de produit.
Point de fusion : 217-220°C.

1.3. Méthanesulfonate de α-aminométhyl-2-méthoxy-5-sulfonamidobenzèneméthanol

Au produit obtenu à l'étape 1.2., on ajoute 1 équivalent d'acide méthanesulfonique en solution 2M dans le méthanol. Après recristallisation dans le méthanol et l'éther diéthylique et séchage au dessicateur sous vide sur anhydride phosphorique, on obtient 0,370 g de produit.
Point de fusion : 210-212°C.

Exemple 2 : méthanesulfonate de (-)-α-(aminométhyl)-2-méthoxy-5-sulfonamidobenzèneméthanol

2.1. 2-Méthoxy-5-chlorosulfonylacétophénone

Dans un ballon de 1 litre, on introduit 951 g (8,16 mol) de chlorhydrine sulfurique. On refroidit vers - 5°C, puis on ajoute, goutte à goutte, 81,5 g (0,544 mol) de 2-méthoxy acétophénone sans dépasser 0°C. On agite ensuite le mélange à température ambiante pendant 16 heures, puis on le verse lentement sur de la glace pilée, tout en agitant. On filtre, on lave le produit à l'eau glacée puis on le sèche au dessicateur sous vide sur anhydride phosphorique. On obtient 87,5 g de produit.
Point de fusion : 85-86°C.

2.2. 2-Méthoxy-5-sulfonamidoacétophénone

Dans un ballon de 1 litre, on introduit 86 g (0,344 mol) de 2-méthoxy-5-chlorosulfonylacétophénone et 690 ml de chloroforme. On agite le mélange jusqu'à dissolution, puis on le refroidit à 0°C par un bain de glace et on fait passer un courant d'ammoniac dans la solution pendant 1 heure. On laisse ensuite le mélange revenir à température ambiante, puis on évapore le solvant et on ajoute 250 ml d'acide chlorhydrique 1M. On agite la suspension obtenue pendant 3 heures, puis on filtre, on lave le produit à l'eau glacée et on le sèche au dessicateur sous vide sur anhydride phosphorique. On obtient 72,8 g de produit.
Point de fusion : 161-162°C.

Selon le même procédé, on a obtenu la 2-méthyl-5-sulfonamido acétophénone.
Point de fusion : 215°C.

2.3. α-Bromo-2-méthoxy-5-sulfonamidoacétophénone

Dans un tricol de 1 litre, on introduit 60,36 g (0,262 mol) de 2-méthoxy-5-sulfonamidoacétophénone et 530 ml d'acide acétique. On agite le mélange et on chauffe à 50 °C. On ajoute ensuite, goutte à goutte, 41,95 g (0,262 mol) de brome, on agite le mélange pendant 16 heures en le laissant revenir à température ambiante et on le filtre. On lave le précipité avec un minimum d'éthanol et on le sèche au dessicateur sous vide sur anhydride phosphorique. On obtient 49 g de produit.
Point de fusion : 154-156°C.

2.4. α-Azido-2-méthoxy-5-sulfonamidoacétophénone

Dans un tricol de 100 ml, on introduit 7 g (0,023 mol) de α-bromo-2-méthoxy-5-sulfonamidoacétophénone, 2,6 ml (0,045 mol) d'acide acétique et 23 ml d'éthanol. On chauffe la suspension à 50°C tout en agitant, puis on ajoute, goutte à goutte, une solution de 2,94 g (0,045 mol) d'azoture de sodium dans 8 ml d'eau. On agite la suspension à 50°C pendant 45 minutes, puis on la laisse revenir à température ambiante. On filtre, on lave le précipité avec un minimum d'éthanol froid, puis on le sèche au dessicateur sous vide sur anhydride phosphorique. On obtient 5,46 g de produit. Point de fusion : 155-160°C (avec décomposition).

2.5. (-)-α-Azidométhyl-2-méthoxy-5-sulfonamidobenzène méthanol

Dans un tricol de 500 ml, on introduit 16,2 g (0,060 mole) de α-azido-2-méthoxy-5-sulfonamidoacétophénone et 240 ml de tétrahydrofuranne anhydre. On refroidit la solution à - 25°C, et on ajoute une solution de 38,5 g (0,12 mol) de (-)-DIP-Cl dans 30 ml de tétrahydrofuranne anhydre, sous un débit de 1,5 ml/mn. Au bout de 90 minutes, on laisse la solution revenir à température ambiante et on ajoute 10 ml de méthanol. On concentre ensuite le mélange réactionnel et on purifie le résidu par chromatographie sur colonne avec un mélange 40/60 d'éther de pétrole et d'acétate d'éthyle. Après recristallisation dans l'isopropanol et séchage au dessicateur sous vide sur anhydride phosphorique, on obtient 11,55 g de produit (ee = 99,9 %).
Point de fusion : 122-125°C.
$[\alpha]_D^{20}$ = - 147,7° (c = 1 ; diméthylsulfoxyde)

2.6. (-)-α-Aminométhyl-2-méthoxy-5-sulfonamidobenzène méthanol

Dans un réacteur de 1 litre, on introduit 11 g (0,040 mol) de (-)-α-azidométhyl-2-méthoxy-5-sulfonamidobenzèneméthanol, 500 ml d'éthanol et 2,2 g de palladium sur charbon à 10 %. On ferme le réacteur, on le purge à l'azote et on agite le mélange sous 400 kPa d'hydrogène, à température ambiante, pendant 3 heures. On filtre ensuite le mélange réactionnel sur papier whatman, on met en suspension le catalyseur récupéré dans 200 ml de méthanol et on chauffe le mélange à ébullition pendant 30 minutes. On filtre ensuite sur papier whatman, on réunit les filtrats, on les concentre et on sèche le résidu au dessicateur sous vide sur anhydride phosphorique. On obtient 9,4 g de (-)-α-aminométhyl-2-méthoxy-5-sulfonamidobenzèneméthanol.
Par recristallisation du produit dans 388 ml de méthanol, on obtient 5,46 g de produit. D'autre part, par concentration des eaux-mères et recristallisation du résidu dans 400 ml d'éthanol, on obtient 1,93 g de produit, soit au total 7,39 g de produit.
Point de fusion : 217-220°C.
$[\alpha]_D^{20}$ = - 85,2° (méthanol).

2.7. Méthanesulfonate de (-)-α-aminométhyl-2-méthoxy-5-sulfonamidobenzèneméthanol

Au produit obtenu à l'étape précédente, on ajoute 1 équivalent d'acide méthanesulfonique en solution 2M dans le méthanol. Après recristallisation dans le méthanol et l'éther diéthylique et séchage du produit au dessicateur sous vide sur anhydride phosphorique, on obtient le méthanesulfonate de (-)-α-aminométhyl-2-méthoxy-5-sulfonamidobenzèneméthanol. Point de fusion : 232-233°C.
$[\alpha]_D^{20}$ = - 41,0° (c = 0,796 ; eau)

<u>Exemple 3</u> : <u>méthanesulfonate de (+) et de (-)-α-aminométhyl-2-méthoxy-5-sulfonamidobenzèneméthanol</u>

3.1. α-Chloro-2-méthoxy-5-sulfonamidoacétophénone

Dans un ballon de 500 ml, on introduit 4,36 g (14,1 mmol) de α-bromo-2-méthoxy-5-sulfonamidoacétophénone, 200 ml d'acétone anhydre et 50 g de chlorure de lithium. On chauffe le mélange à reflux pendant 16 heures, puis on concentre la solution, on ajoute 200 ml d'eau et on extrait avec 3 fois 80 ml d'acétate d'éthyle. On réunit les phases organiques, on les sèche sur sulfate de magnésium et on les concentre. On obtient 3,56 g de produit.
Point de fusion : 162°C.

3.2. α-Chlorométhyl-2-méthoxy-5-sulfonamidobenzèneméthanol

Dans un ballon de 100 ml, on introduit 10 ml de tétrahydrofuranne anhydre et 4 ml d'une solution 1M de borane dans le tétrahydrofuranne. On ajoute, goutte à goutte, une solution de 1,0 g (3,8 mmol) de α-chloro-2-méthoxy-5-sulfonamido acétophénone dans 10 ml de tétrahydrofuranne. On agite le mélange pendant 10 heures à température ambiante, puis on ajoute 10 ml de méthanol. On concentre la solution, on ajoute 40 ml d'eau et on extrait avec 3 fois 60 ml d'acétate d'éthyle. On réunit les phases organiques, on les sèche sur sulfate de magnésium et on les concentre. On obtient 1,0 g de produit.
Point de fusion : 112°C.

3.3. Acétate de (±)-α-chlorométhyl-2-méthoxy-5-sulfonamido benzyle

Dans un ballon de 250 ml, on introduit 2,64 g (9,9 mmol) de α-chlorométhyl-2-méthoxy-5-sulfonamidobenzèneméthanol et 100 ml de dichlorométhane. On ajoute ensuite, sous agitation, 10 ml de diméthylformamide, puis 852 µl d'acide acétique, 2,56 g de dicyclohexylcarbodiimide et 121 mg de diméthylaminopyridine. On laisse réagir le mélange pendant 1 heure à température ambiante, puis on le filtre, on le rince avec 50 ml d'une solution d'hydrogénocarbonate de sodium à 5 %, puis avec 50 ml d'eau. On extrait les eaux de rinçage avec 2 ois 20 ml d'acide acétique, puis on réunit les phases organiques, on les sèche et on les concentre. Par chromatographie du résidu sur colonne de silice, avec un mélange 25/75 d'acétate d'éthyle et de cyclohexane, on obtient 1,9 g de produit.
Point de fusion : 131°C.

3.4. (+)- et (-)-α-Chlorométhyl-2-méthoxy-5-sulfonamido benzèneméthanol

Dans un tricol de 500 ml, on introduit 2,86 g (9,3 mmol) d'acétate de (±)-α-chlorométhyl-2-méthoxy-5-sulfonamido benzyle et 110 ml de t-butyl-méthyléther. On agite pendant 15 minutes, puis on ajoute 170 ml de tampon phosphate et on agite fortement jusqu'à obtention d'une émulsion. On ajoute alors 0,57 g (20 %) de lipase SP 523 et on suit la réaction à température ambiante, par pHstat (addition d'hydroxyde de sodium 1M) et par HPLC sur colonne chirale et on détermine le taux de conversion de l'ester et les excès énantiomériques de l'ester et de l'alcool. Au bout de 45 heures de réaction, les excès énantiomériques de l'ester et de l'alcool étant supérieurs à 95 %, on dilue le milieu réctionnel avec 800 ml d'acétate d'éthyle, on sépare la phase organique et on réextrait la phase aqueuse avec 3 fois 500 ml d'acétate d'éthyle. On réunit les phases organiques, on les sèche, on les concentre et on purifie le résidu par 2 chromatographies flash successives sur colonne de silice avec un mélange 30/70 d'acétate d'éthyle et de cyclohexane. On obtient 1,32 g d'acétate de (-)-α-chlorométhyl-2-méthoxy-5-sulfonamido benzyle (ee = 99 %) et 1,05 g de (+)-α-chlorométhyl-2-méthoxy-5-sulfonamidobenzèneméthanol.
L'énantiomère (+) est purifié par dissolution dans 10 ml d'acétate d'éthyle et recristallisation par addition d'hexane (ee : 98 %).
Point de fusion : 117-118°C.
$[\alpha]_D^{20}$ = + 40° (c = 0,305 ; méthanol).

On ajoute 61 µl de chlorure d'acétyle à 100 ml de méthanol et on agite le mélange pendant 15 minutes. On ajoute alors 1,32 g d'acétate de (-)-α-chlorométhyl-2-méthoxy-5-sulfonamidobenzyle et on chauffe à reflux pendant 1 heure (taux de conversion de 97 % indiqué par HPLC). On évapore ensuite le mélange puis on reprend le résidu dans 100 ml d'acétate d'éthyle et on neutralise le milieu par 5 ml d'hydrogénocarbonate d'éthyle à 2 %. On extrait la phase carbonate avec 2 fois 5 ml d'acétate d'éthyle, puis on réunit les phases organiques, on les sèche et on les concentre jusqu'à 30 ml et on ajoute du cyclohexane. Au bout d'une nuit à température ambiante, le produit cristallisé est filtré. On obtient 1 g de (-)-α-chlorométhyl-2-méthoxy-5-sulfonamido benzèneméthanol.
Point de fusion : 114-115°C.
$[\alpha]_D^{20}$ = - 41,4° (c = 0,295 ; méthanol).

3.5. (+)-α-Azidométhyl-2-méthoxy-5-sulfonamidobenzène méthanol

Dans un ballon de 250 ml, on introduit 1,58 g (5,9 mmol) de (+)-α-chlorométhyl-2-méthoxy-5-sulfonamidobenzèneméthanol, 20 ml de diméthylformamide et 1,54 g d'azoture de sodium. On chauffe le mélange réactionnel à 110°C pendant 16 heures, puis on ajoute 200 ml d'eau et on extrait avec 3 fois 80 ml d'acétate d'éthyle. On réunit ensuite les phases organiques, on les sèche sur sulfate de magnésium et on les concentre. On obtient 1,2 g de produit.
Point de fusion : 122°C.
$[\alpha]_D^{20}$ = + 144° (c = 1 ; diméthylsulfoxyde).

Selon le même procédé, à partir du (-)-α-chlorométhyl-2-méthoxy-5-sulfonamidobenzèneméthanol, on a obtenu le (-)-α-azidométhyl-2-méthoxy-5-sulfonamidobenzène méthanol.
Point de fusion : 122°C.
$[\alpha]_D^{20}$ = - 147,7° (c= 1 ; diméthylsulfoxyde)

3.6. (+)-α-Aminométhyl-2-méthoxy-5-sulfonamidobenzèneméthanol

A partir du (+)-α-azidométhyl-2-méthoxy-5-sulfonamidobenzène méthanol traité dans les conditions décrites à l'étape 6 de l'exemple 2, on a obtenu le (+)-α-aminométhyl-2-méthoxy-5-sulfonamidobenzèneméthanol.
Point de fusion : 217-220°C.
$[\alpha]_D^{20}$ = + 40° (c = 1 ; diméthylsulfoxyde).
Selon le même procédé, à partir du (-)-α-azidométhyl-2-méthoxy-5-sulfonamidobenzèneméthanol, on a obtenu le (-)-α-aminométhyl-2-méthoxy-5-sulfonamidobenzèneméthanol.
Point de fusion : 217-220°C.
$[\alpha]_D^{20}$ = - 44° (c = 1 ; diméthylsulfoxyde).

3.7. Méthanesulfonate de (+)-α-aminométhyl-2-méthoxy-5-sulfonamidobenzèneméthanol

A partir du (+)-α-aminométhyl-2-méthoxy-5-sulfonamidobenzène méthanol traité par 1 équivalent d'acide méthanesulfonique en solution 2M dans le méthanol, recristallisation dans le méthanol et l'éther diéthylique et séchage au dessicateur sous vide sur anhydride phosphorique, on a obtenu le méthanesulfonate de (+)-α-aminométhyl-2-méthoxy-5-sulfonamidobenzèneméthanol.
Point de fusion : 234°C.
$[\alpha]_D^{20}$ = + 41° (c = 0,9945 ; eau).
Selon le même procédé, à partir (-)-α-aminométhyl-2-méthoxy-5-sulfonamidobenzèneméthanol, on a obtenu le méthanesulfonate de (-)-α-aminométhyl-2-méthoxy-5-sulfonamidobenzèneméthanol.
Point de fusion : 235°C.
$[\alpha]_D^{20}$ = - 37,3°(c = 0,969 ; méthanol/eau 80/20)

<u>Exemple 4</u> : <u>méthanesulfonate de (+) et de (-)-α-aminométhyl-2-méthoxy-5-sulfonamidobenzèneméthanol</u>

4.1. (±) -α-Azidométhyl-2-méthoxy-5-sulfonamidobenzène méthanol

Dans un ballon de 500 ml, on introduit 5 g (18,5 mmol) de α-azido-2-méthoxy-5-sulfonamidoacétophénone et 150 ml de méthanol. On refroidit la solution à 0°C, puis on ajoute 0,963 g (16,6 mmol) de borohydrure de sodium. On agite la solution pendant 10 minutes puis on la laisse revenir à température ambiante et on ajoute 15 ml d'une solution d'acide chlorhydrique à 5 %. On concentre ensuite le mélange réactionnel, puis on purifie le résidu par chromatographie sur colonne avec un mélange 40/60 d'éther de pétrole et d'acétate d'éthyle et on le sèche au dessicateur sous vide sur anhydride phosphorique.
On obtient 3,85 g de produit.
Point de fusion : 123°C.

4.2. (+)- et (-)-α-Azidométhyl-2-méthoxy-5-sulfonamido benzèneméthanol, N-carbobenzyloxy-L-alanine ester

Dans un ballon de 250 ml, on introduit 4,66 g (20,9 mmol) de N-carbobenzyloxy-L-alanine, 25 ml de dichlorométhane et 3,58 g (17,4 mmol) de 1,3-dicyclohexylcarbodiimide. On agite le mélange pendant 20 minutes à température ambiante, puis on ajoute 3,8 g (13,9 mmol) de α-azidométhyl-2-méthoxy-5-sulfonamidobenzèneméthanol et 0,17 g (0,14 mmol) de diméthylaminopyridine. On agite le milieu réactionnel pendant 2 heures, puis on le concentre sous vide et on purifie le résidu par plusieurs chromatographies sur colonne de silice avec un mélange 99/1 de dichlorométhane et d'acétone. On obtient 1,58 g de (+)-α-azidométhyl-2-méthoxy-5-sulfonamido benzèneméthanol, N-carbobenzyloxy-L-alanine ester et 2,92 g de (-)-α-azidométhyl-2-méthoxy-5-sulfonamidobenzèneméthanol, N-carbobenzyloxy-L-alanine ester.
Point de fusion : 170°C (avec décomposition).

4.3. (+)-Azidométhyl-2-méthoxy-5-sulfonamidobenzèneméthanol

Dans un ballon de 100 ml, on introduit 0,91 g (1,9 mmol) de (+) -α-azidométhyl-2-méthoxy-5-sulfonamidobenzèneméthanol, N-carbobenzyloxy-L-alanine ester, 20 ml d'éthanol et 3 ml d'une solution 1M d'hydroxyde potassium dans un mélange 1/1 d'éthanol et d'eau. On agite le mélange réactionnel pendant 25 minutes à température ambiante, puis on le concentre sous vide et on purifie le résidu par chromatographie sur colonne avec un mélange 95/5 de dichlorométhane et de méthanol.
On obtient 0,41 g de produit.
Point de fusion : 122°C.

Selon le même procédé, à partir du (-)-α-azidométhyl-2-méthoxy-5-sulfonamidobenzèneméthanol, N-carbobenzyloxy-L-alanine ester, on a obtenu le (-)-α-azidométhyl-2-méthoxy-5-sulfonamidobenzèneméthanol.
Point de fusion : 122°C.

4.4. Méthanesulfonate de (+)-α-Aminométhyl-2-méthoxy-5-sulfonamidobenzèneméthanol

A partir du (+)-α-azidométhyl-2-méthoxy-5-sulfonamidobenzène méthanol hydrogéné dans les conditions décrites à l'étape 6 de l'exemple 2, puis traité par 1 équivalent d'acide méthanesulfonique, on a obtenu le méthanesulfonate de (+)-α-amino méthyl-2-méthoxy-5-sulfonamidobenzèneméthanol.
Point de fusion : 235°C.
$[\alpha]_D^{20}$ = + 35° (c = 1 ; méthanol/eau 80/20)
Selon le même procédé, à partir du (-)-α-azidométhyl-2-méthoxy-5-sulfonamidobenzèneméthanol, on a obtenu le méthanesulfonate de (-)-α-aminométhyl-2-méthoxy-5-sulfonamidobenzèneméthanol.
Point de fusion : 233°C.
$[\alpha]_D^{20}$ = - 41,8° (c = 1 ; méthanol/eau 80/20)

Exemple 5 : méthanesulfonate de α-diéthylaminométhyl-2-méthoxy-5-sulfonamidobenzèneméthanol

5.1. Oxyde de 2-méthoxy-5-sulfonamidostyrène

Dans un ballon de 100 ml, on introduit 2 g (6,5 mmol) de α-bromo-2-méthoxy-5-sulfonamidoacétophénone, 20 ml d'éthanol anhydre et 1,0 g (7,0 mmol) de carbonate de potassium. On ajoute ensuite 0,41 g (10,8 mmol) de borohydrure de sodium, on agite le mélange à température ambiante pendant 20 minutes, puis on ajoute 5 ml d'une solution 0,1M d'hydroxyde de sodium et on agite pendant 30 minutes. On concentre la solution, on ajoute 30 ml d'eau et on extrait avec 3 fois 30 ml d'acétate d'éthyle. On réunit les phases organiques, on les sèche sur sulfate de magnésium et on les concentre. On obtient 1,41 g de produit.
Point de fusion : 118°C.

5.2. Méthanesulfonate de α-diéthylaminométhyl-2-méthoxy-5-sulfonamidobenzèneméthanol

Dans un ballon de 100 ml, on introduit 1,41 g (6,1 mmol) d'oxyde de 2-méthoxy-5-sulfonamidostyrène, 10 ml d'éthanol anhydre et 17,8 g (244 mmol) de diéthylamine. On chauffe le mélange à reflux sous agitation pendant 16 heures, puis on concentre la solution. On purifie le résidu par chromatographie sur colonne avec un mélange 90/9/1 de dichlorométhane, de méthanol et d'ammoniaque puis on le sèche au dessicateur sous vide sur anhydride phosphorique. On obtient 1,42 g de produit sous forme d'huile, qui est traité par 1 équivalent d'acide méthanesulfonique en solution 2M dans le méthanol. Après recristallisation dans le méthanol et l'éther diéthylique et séchage au dessicateur sous vide sur anhydride phosphorique, on obtient 0,875 g de méthanesulfonate de α-diéthylaminométhyl-2-méthoxy-5-sulfonamidobenzèneméthanol.
Point de fusion : 90-92°C.

Exemple 6 : méthanesulfonate de α-méthylaminométhyl-2-méthoxy-5-sulfonamidobenzèneméthanol

6.1. α-Benzylméthylaminométhyl-2-méthoxy-5-sulfonamido benzèneméthanol

L'oxyde de 2-méthoxy-5-sulfonamidostyrène obtenu à l'étape 1 de l'exemple 4, traité par la benzylméthylamine, dans les conditions décrites à l'étape 2 de l'exemple 4, donne le α-benzylméthylaminométhyl-2-méthoxy-5-sulfonamidobenzène méthanol sous forme d'huile.

6.2. Méthanesulfonate de α-méthylaminométhyl-2-méthoxy-5-sulfonamidobenzèneméthanol

Par hydrogénation de 1.90 g (5,4 mmol) de α-benzylméthyl aminométhyl-2-méthoxy-5-sulfonamidobenzène méthanol dans les conditions décrites à l'étape 6 de l'exemple 2, on obtient le α-méthylaminométhyl-2-méthoxy-5-sulfonamidobenzèneméthanol. Après recristallisation dans l'acétate d'éthyle et le méthanol, on traite le produit obtenu par 1 équivalent d'acide méthanesulfonique en solution 2 M dans le méthanol et on recristallise le sel à partir de méthanol, de dichlorométhane et d'éther diéthylique. On obtient ainsi 0,396 g de méthanesulfonate de α-méthylaminométhyl-2-méthoxy-5-sulfonamidobenzèneméthanol.
Point de fusion : 194-196°C.

Exemple 7 : méthanesulfonate de α-aminométhyl-2-fluoro-5-sulfonamidobenzèneméthanol

7.1. 2-Fluoro-5-nitroacétophénone

Dans un tricol de 100 ml contenant 60 ml d'acide sulfurique concentré, refroidi à - 5°C, on introduit, goutte à goutte, 25 ml (180 mmol) de 2-fluoroacétophénone. On ajoute ensuite, goutte à goutte, un mélange de 14 ml d'acide nitrique (d = 1,42) et de 20 ml d'acide sulfurique concentré, sans dépasser 0°C. On agite le mélange à - 5°C pendant 30 minutes puis on le verse sur de la glace pilée. On extrait ensuite avec 3 fois 60 ml d'acétate d'éthyle, puis on réunit les phases organiques, on les sèche sur sulfate de magnésium et on les concentre. On purifie le résidu par chromatographie sur colonne avec un mélange 70/30 d'hexane et d'acétate d'éthyle, puis on le sèche au dessicateur sous vide sur anhydride phosphorique. On obtient 26 g de produit.
Point de fusion : 72°C.

Selon le même procédé, on a obtenu les composés suivants :

- la 2-chloro-5-nitroacétophénone.
  Point de fusion : 65°C.
- la 2-hydroxy-5-nitroacétophénone.
  Point de fusion : 98°C,

qui est convertie, par réaction de catalyse de tranfert de phase avec l'iodure d'isopropyle, en 2-isopropoxy-5-nitro acétophénone,
Point de fusion : 78°C.

7.2. 5-Amino-2-fluoroacétophénone

Dans un tricol de 1 litre, on introduit 25,4 g (152 mmol) de 2-fluoro-5-nitroacétophénone, 343 g (1,52 mol) de chlorure d'étain dihydrate et 250 ml d'acétate d'éthyle. On chauffe le mélange réactionnel à 70°C pendant 30 minutes puis on le verse sur 1 litre de glace pilée, on ajoute une solution d'hydroxyde de sodium à 30 % et on extrait avec 3 fois 350 ml d'acétate d'éthyle. On réunit les phases organiques, on les sèche sur sulfate de magnésium et on les concentre. On obtient 11,26 g de produit sous forme d'huile.

Selon le même procédé, on a obtenu les composés suivants :

- la 5-amino-2-chloroacétophénone, sous forme d'huile.
- la 5-amino-2-isopropoxyacétophénone, sous forme d'huile.

7.3. 2-Fluoro-5-sulfonamidoacétophénone

Dans un tricol de 250 ml, on introduit 15,3 g (100 mmol) de 5-amino-2-fluoroacétophénone et 50 ml d'acide acétique, puis on ajoute 50 ml d'acide chlorhydrique concentré. On refroidit le mélange réactionnel à 0°C, puis on ajoute, goutte à goutte, une solution de 10,3 g (150 mmol) de nitrite de sodium dans 25 ml d'eau et on laisse à 0°C pendant 30 minutes. On ajoute alors une suspension, refroidie à - 15°C, de 5 g (29 mmol) de dihydrate de chlorure cuivreux et 30 g (470 mmol) d'anhydride sulfureux dans 75 ml d'acide acétique. On maintient le mélange à 0°C pendant 48 heures, puis on ajoute 20 ml d'eau, on extrait avec 3 fois 120 ml de dichlorométhane, puis on réunit les phases organiques, on les sèche sur sulfate de magnésium et on les concentre. On dissout le résidu dans 100 ml de tétrahydrofuranne, puis on ajoute, goutte à goutte, à 0°C, une solution d'ammoniaque à 28 %. On agite le mélange réactionnel pendant 16 heures à température ambiante puis on le concentre. On purifie le résidu par chromatographie sur colonne avec un mélange 60/40 d'hexane et d'acétate d'éthyle et on le sèche au dessicateur sous vide sur anhydride phosphorique. On obtient 11,23 g de produit.
Point de fusion : 112°C.

Selon le même procédé, on a obtenu les composés suivants :

- la 2-chloro-5-sulfonamidoacétophénone.
  Point de fusion : 106°C.
- la 2-isopropoxy-5-sulfonamidoacétophénone.
  Point de fusion : 85°C.
- la 3-sulfonamidoacétophénone.
  Point de fusion : 144°C.

7.4. α-Bromo-2-fluoro-5-sulfonamidoacétophénone

A partir de la 2-fluoro-5-sulfonamidoacétophénone traitée dans les conditions décrites à l'étape 3 de l'exemple 2, on a obtenu la α-bromo-2-fluoro-5-sulfonamidoacétophénone. Point de fusion : 122°C.

Selon le même procédé, on a obtenu les composés suivants :

- la α-bromo-2-chloro-5-sulfonamidoacétophénone. Point de fusion : 126°C.
- la α-bromo-2-isopropoxy-5-sulfonamidoacétophénone. Point de fusion : 105°C.
- la α-bromo-3-sulfonamidoacétophénone Point de fusion : 130°C.

7.5. α-Chloro-2-fluoro-5-sulfonamidoacétophénone

A partir de la α-bromo-2-fluoro-5-sulfonamidoacétophénone traitée dans les conditions décrites à l'étape 1 de l'exemple 3, on a obtenu la α-chloro-2-fluoro-5-sulfonamido acétophénone.
Point de fusion : 114°C.

Selon le même procédé, on a obtenu les composés suivants :

- la α-chloro-2-chloro-5-sulfonamidoacétophénone. Point de fusion : 124°C.
- la α-chloro-2-isopropoxy-5-sulfonamidoacétophénone. Point de fusion : 98°C.
- la α-chloro-3-sulfonamidoacétophénone. Point de fusion : 128°C.

7.6. α-Chlorométhyl-2-fluoro-5-sulfonamidobenzèneméthanol

A partir de la α-chloro-2-fluoro-5-sulfonamidoacétophénone traitée dans les conditions de l'étape 2 de l'exemple 3, on a obtenu le α-chlorométhyl-2-fluoro-5-sulfonamidobenzène méthanol.
Point de fusion : 112°C.

Selon le même procédé, on a obtenu les composés suivants :

- le α-chlorométhyl-2-chloro-5-sulfonamidobenzèneméthanol. Point de fusion : 115°C.
- le α-chlorométhyl-2-isopropoxy-5-sulfonamidobenzène méthanol. Point de fusion : 93°C.
- le α-chlorométhyl-3-sulfonamidobenzèneméthanol. Point de fusion : 122°C.

7.7. α-Azidométhyl-2-fluoro-5-sulfonamidobenzèneméthanol

A partir du α-chlorométhyl-2-fluoro-5-sulfonamidobenzène méthanol, traité dans les conditions décrites à l'étape 4 de l'exemple 3, on a obtenu le α-azidométhyl-2-fluoro-5-sulfonamidobenzèneméthanol.
Point de fusion : 86°C.

Selon le même procédé, on a obtenu les composé suivants :

- le α-azidométhyl-2-chloro-5-sulfonamidobenzèneméthanol. Point de fusion : 122°C.
- le α-azidométhyl-2-isopropoxy-5-sulfonamidobenzèneméthanol. Point de fusion : 95°C.
- le α-azidométhyl-3-sulfonamidobenzèneméthanol. Point de fusion : 118°C.

7.8. Méthanesulfonate de α-aminométhyl-2-fluoro-5-sulfonamidobenzèneméthanol

A partir du α-azidométhyl-2-fluoro-5-sulfonamidobenzène méthanol traité dans les conditions de l'étape 6 de l'exemple 2, on a obtenu le méthanesulfonate de α-aminométhyl-2-fluoro-5-sulfonamidobenzèneméthanol.
Point de fusion : 164°C.

Exemple 8 : méthanesulfonate de α-aminométhyl-2-méthyl-5-sulfonamidobenzèneméthanol

8.1. α-Chloro-2-méthyl-5-sulfonamidoacétophénone

Dans un ballon de 500 ml, on introduit 26,4 g (76,0 mmol) de dichloroiodate de benzyltriméthylammonium (préparé selon la méthode décrite dans Synthesis 7, (1988), 545), 9,25 g (43,4 mmol) de 2-méthyl-5-sulfonamidoacétophénone, 90 ml de méthanol et 220 ml de 1,2-dichloroéthane. On chauffe à reflux pendant 16 heures, puis on concentre le milieu réactionnel et on ajoute 200 ml d'une solution saturée de bicarbonate de sodium. On extrait avec 3 fois 120 ml d'acétate d'éthyle, puis on réunit les phases organiques, on les sèche sur sulfate de magnésium et on les concentre. On purifie le résidu par chromatographie sur colonne avec un mélange 60/40 d'hexane et d'acétate d'éthyle et on le sèche au dessicateur sous vide sur anhydride phosphorique. On obtient 1,7 g de produit.
Point de fusion : 114°C.

8.2. α-Chlorométhyl-2-méthyl-5-sulfonamidobenzèneméthanol

A partir de la α-chloro-2-méthyl-5-sulfonamidoacétophénone traitée dans les conditions de l'étape 2 de l'exemple 3, on a obtenu le α-chlorométhyl-2-méthyl-5-sulfonamidobenzène méthanol.
Point de fusion : 126°C.

8.3. α-Azidométhyl-2-méthyl-5-sulfonamidobenzèneméthanol

A partir du α-chlorométhyl-2-méthyl-5-sulfonamidobenzène méthanol traité dans les conditions décrites à l'étape 4 de l'exemple 3, on a obtenu le α-azidométhyl-2-méthyl-5-sulfonamidobenzèneméthanol.
Point de fusion : 98°C.

8.4. Méthanesulfonate de α-aminométhyl-2-méthyl-5-sulfonamidobenzèneméthanol

A partir du α-azidométhyl-2-méthyl-5-sulfonamidobenzène méthanol traité dans les conditions décrites à l'étape 6 de l'exemple 2, on a obtenu le méthanesulfonate de α-amino méthyl-2-méthyl-5-sulfonamidobenzèneméthanol.
Point de fusion : 185°C.

Exemple 9 : Méthanesulfonate de α-aminométhyl-2-chloro-5-sulfonamidobenzèneméthanol

Dans un ballon de 250 ml, on introduit 1,35 g (4,9 mmol) de α-azidométhyl-2-chloro-5-sulfonamidobenzèneméthanol, 90 ml de pyridine anhydre et 9,67 g (29,0 mmol) de triphénylphosphine sur support. On agite le mélange à température ambiante pendant 9 heures, puis on ajoute 100 ml d'ammoniaque à 28 %, on agite la suspension pendant 16 heures et on filtre. On concentre le filtrat et on recristallise le résidu dans le méthanol. On obtient 0,523 g de α-aminométhyl-2-chloro-5-sulfonamidobenzèneméthanol. On ajoute 1 équivalent d'acide méthanesulfonique en solution 2M dans le méthanol. Après recristallisation dans le méthanol et l'éther diéthylique et séchage au dessicateur sous vide sur anhydride phosphorique, on obtient 0,439 g de méthanesulfonate de α-aminométhyl-2-chloro-5-sulfonamidobenzèneméthanol.
Point de fusion : 206-208°C.

Exemple 10 : Syn et anti (2'-méthoxy-5'-aminosulfonyl)-phenyl-2-amino-propan-1-ol

10.1. 2-Méthoxy-5-chlorosulfonylpropiophénone

A partir de la 2-méthoxy-propiophénone traitée dans les conditions de l'étape 1 de l'exemple 2, on a obtenu la 2-méthoxy-5-chlorosulfonylpropiophénone.
Point de fusion : 86-89°C

10.2. 2-Méthoxy-5-sulfonamidopropiophénone

A partir de la 2-méthoxy-5-chlorosulfonylpropiophénone traitée dans les conditions de l'étape 2 de l'exemple 2, on a obtenu la 2-méthoxy-5-sulfonamidopropiophénone.
Point de fusion : 162-165°C

10.3. 2-Bromo-2'-méthoxy-5'-sulfonamidopropiophénone

A partir de la 2-méthoxy-5-sulfonamidopropiophénone traitée dans les conditions de l'étape 3 de l'exemple 2, on a obtenu la 2-bromo-2'-méthoxy-5'-sulfonamidopropiophénone.
Point de fusion : 108-110°C

10.4. 2-Azido-2'-méthoxy-5'-sulfonamidopropiophénone.

A partir de la α-bromo-2-méthoxy-5-sulfonamido propiophénone traitée dans les conditions de l'étape 4 de l'exemple 2, on a obtenu la 2-azido-2'-méthoxy-5'-sulfonamidopropiophénone. Point de fusion : 113-114°C

10.5. (2'-Méthoxy-5'-aminosulfonyl)-phényl-2-azidopropan-1-ol

A partir de la 2-azido-2'-méthoxy-5'-sulfonamido propiophénone traitée dans les conditions de l'étape 1 de l'exemple 4, on a obtenu la (2'-méthoxy-5'-aminosulfonyl)phényl-2-azidopropan-1-ol.
Point de fusion : 109-110°C

10.6. Syn et Anti (2'-méthoxy-5'-aminosulfonyl)-phényl-2-aminopropan-1-ol.

A partir de la (2'-méthoxy-5'-aminosulfonyl)-phényl-2-azidopropan-1-ol traitée dans les conditions de l'étape 6 de l'exemple 2, on a obtenu un mélange de syn et anti (2'-méthoxy-5'-aminosulfonyl)-phényl-2-aminopropan-1-ol, qui sont séparés par de successives chromatographies sur colonne de silice avec un solvant d'élution dichlorométhane : méthanol : ammoniac 95 : 5 : 0,5 pour fournir les diastéréoisomères syn et anti.
Après recristallisation dans l'isopropanol et séchage du dessiccateur sous vide sur anhydride phosphorique, on obtient le syn (2'-méthoxy-5'-aminosulfonyl)-phényl-2-aminopropan-1-ol.
Point de fusion : 176-177°C et l'anti (2'-méthoxy-5'-aminosulfonyl)-phényl-2-aminopropan-1-ol.
Point de fusion : 233-237°C

<u>Exemple 11</u> : <u>(-)-Syn-(2'-méthoxy-5'-sulfonamido)-phényl-2-aminopropan-1-ol</u>

11.1. (-)-Syn-(2'-méthoxy-5'-sulfonamido)-phényl-2-azidopropan-1-ol

A partir de la 2-azido-2'-méthoxy-5'-sulfonamidopropiophénone traitée dans les conditions de l'étape 5 de l'exemple 2, on a obtenu après 2 recristallisations dans l'isopropanol, la (-)-syn-(2'-méthoxy-5'-sulfonamido)-phényl-2-aminopropan-1-ol. Point de fusion : 143-145°C
$[\alpha]^{20}_{D}$ = -125° (méthanol)

11.2. (-)-Syn-(2'-méthoxy-5'-sulfonamido)phényl-2-aminopropan-1-ol.

A partir de la (-)-syn-(2'-méthoxy-5'-aminosulfonyl)-phényl-2-azidopropan-1-ol traitée dans les conditions de l'étape 6 de l'exemple 2, on a obtenu après 2 recristallisations dans l'isopropanol, la (-)-syn-(2'méthoxy-5'-sulfonamidophényl-2-aminopropan-1-ol.
Point de fusion : 190-191°C
$[\alpha]^{20}_{D}$ = -34.1° (méthanol)

Les composés de l'invention sont rassemblés dans le tableau suivant avec leurs caractéristiques physiques.

Tableau

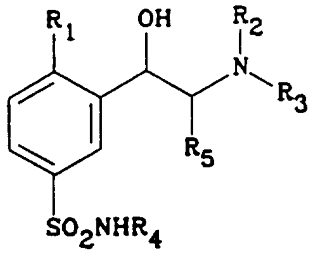

(I)

| N° | R1 | R2 | R3 | R4 | R5 | base/sel | F (°C) |
|---|---|---|---|---|---|---|---|
| 1 | H | H | H | H | H | MeSO3H | 156-161 |
| 2 | Me | H | H | H | H | MeSO3H | 185 |
| 3 (±) | OMe | H | H | H | H | base<br>MeSO3H | 217-220<br>210-212 |
| 4 (+) | OMe | H | H | H | H | base<br>MeSO3H | 217-220<br>234 |
| 5 (-) | OMe | H | H | H | H | base<br>MeSO3H | 217-220<br>235 |
| 6 | O-iPr | H | H | H | H | MeSO3H | 92 |
| 7 | Cl | H | H | H | H | MeSO3H | 206-208 |
| 8 | F | H | H | H | H | MeSO3H | 164 |
| 9 | OMe | Me | H | H | H | MeSO3H | 194-196 |
| 10 | OMe | 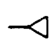 | H | H | H | MeSO3H | 231 |
| 11 | OMe | Et | Et | H | H | MeSO3H | 90-92 |
| 12 | OMe | H | H | Me | H | oxalate | 154-156 |
| 13 | OMe | H | H | 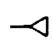 | H | oxalate | 199-202 |
| 14 | OMe | H | H | i-Pr | H | oxalate | 180 |
| 15 | OMe | H | H | t-Bu | H | oxalate | 203-205 |
| 16 anti | OMe | H | H | H | $CH_3$ | Base | 233-237 |
| 17 syn | OMe | H | H | H | $CH_3$ | base | 176-177 |
| 18 (-) syn | OMe | H | H | H | $CH_3$ | base | 143-145 |

MeSO3H représente méthanesulfonate

OMe représente méthoxy

O-iPr représente isopropoxy

i-Pr représente isopropyle

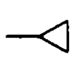 représente cyclopropyle

Les composés de l'invention ont été soumis à des tests biologiques destinés à mettre en évidence leur activité agoniste des récepteurs $\alpha_1$-adrénergiques.

Ils ont en particulier été soumis à des tests de liaison aux sous-récepteurs $\alpha_{1a}$, $\alpha_{1b}$ et $\alpha_{1d}$, réalisés respectivement sur du tissu de glande salivaire de rat, sur du tissu de foie de rat et sur des cellules CHO transfectées.
L'affinité pour chaque type de sous-récepteur, exprimée par la $CI_{50}$ (concentration inhibant de 50 % la liaison à la [$^3$*H*] prazosine), a été déterminée et les valeurs relatives de l'affinité pour le récepteur $\alpha_{1a}$ par rapport aux affinités pour les récepteurs $\alpha_{1b}$ et $\alpha_{1c}$, exprimées par les rapports des $CI_{50}$, [$\alpha_{1b}/\alpha_{1a}$] et [$\alpha_{1d}/\alpha_{1a}$], ont été calculées.
Pour les composés de l'invention, ces rapports varient respectivement de 9,3 à 21,6 et de 7,8 à 20,9, indiquant une sélectivité importante pour le récepteur $\alpha_{1a}$.

L'activité *in vitro* des composés de l'invention a été étudiée sur les muscles lisses urétraux et artériels.
Ces essais ont été réalisés sur des lapins femelles néo-zélandais pesant de 3 à 3,5 kg. Les animaux ont été tués par dislocation vertébrale puis on a prélevé des anneaux de tissu d'artères mésentériques et d'urètre. Ces anneaux de tissu ont été immergés dans une solution de Krebs modifiée, oxygénée par un mélange de 95 % de $O_2$ et 5 % de $CO_2$.
Chaque échantillon de tissu a été soumis à une tension de 1 g puis on a introduit la phényléphrine à des doses cumulatives et établi la courbe dose/réponse. Après rincage des échantillons, on a introduit le composé à étudier à des doses cumulatives et établi la courbe dose/réponse. L'effet $\alpha_1$-adrénergique de chaque composé est évalué par le calcul du $pD_2$ (logarithme négatif de la concentration d'antagoniste en présence duquel l'effet d'une dose de l'agoniste est divisée par 2) ainsi que par l'effet maximum représentant le pourcentage de la contraction maximum obtenue avec la phényléphrine (% $E_{max}$).

Pour les composés de l'invention, les $pD_2$ urétraux et artériels varient respectivement entre 4,18 et 4,93 ($pD_2$ phényléphrine = 5,2-5,5) et entre 3,73 et 4,55 ($pD_2$ phényléphrine = 5,2-5,5) et les % $E_{max}$ urétraux et artériels varient respectivement entre 58,4 et 76 et entre 76 et 94,6.

L'activité *in vivo* des composés de l'invention sur la pression sanguine et urétrale a été étudiée chez le lapin.

Ces essais ont été réalisés sur des lapins femelles néo-zélandais, pesant de 3 à 4 kg. Après anesthésie au pentobarbital, des cathéters ont été introduits dans l'aorte abdominale, par l'artère fémorale, dans une veine jugulaire et dans l'urètre (1 cm au dessous du col vésical). Les composés à étudier ont été administrés 5 à 15 jours après l'opération, soit par voie intraveineuse, soit par voie orale.

Par voie intraveineuse, les composés ont été administrés en 5 minutes, en une seule dose, ou en mode cumulatif, à intervalles de 15 minutes entre chaque dose, à des doses de 3 à 100 μg/kg.
La pression sanguine (PA) et la pression urétrale (PU) ont été mesurées en continu pour chaque dose.
Pour les composés de l'invention, l'augmentation des PA est est d'environ 5 mmHg à la dose de 10 μg/kg et 15 mmHg à la dose de 100 μg/kg, l'augmentation des PU est d'environ 14 $cmH_2O$ à la dose de 10 μg/kg et 54 $cmH_2O$ à la dose de 100 μg/kg.

Aux différentes doses testées, les composés de l'invention présentent une forte urosélectivité puisqu'ils augmentent de façon très importante la pression urétrale sans modifier de façon sensible la pression artérielle.

Par voie orale, les composés ont été administrés par gavage en une seule dose de 300 et 1000 μg/kg, sous un volume de 1 ml/kg. Les PA et PU ont été mesurées 5, 10, 30, 45 et 60 minutes après le gavage.

Pour les composés de l'invention, les variations des PA sont respectivement d'environ - 0,2 et - 0,9 mmHg aux doses de 300 et 1000 μg/kg, au bout de 30 minutes, et d'environ - 5,3 et 1,1 mmHg au bout de 60 minutes et les variations des PU sont respectivement d'environ 1,6 et 7,8 $cmH_2O$ aux doses de 300 et 1000 μg/kg, au bout de 30 minutes, et d'environ 3,7 et 8,3 $cmH_2O$ au bout de 60 minutes.

Par voie orale, les composés de l'invention présentent une urosélectivité totale puisque la pression urétrale est augmentée de façon sensible sans que la pression artérielle soit modifiée.

L'ensemble des résultats obtenus montrent que les composés de l'invention ont une forte action urétrale et une faible action artérielle. Ce sont des agonistes des récepteurs $\alpha_1$-adrénergiques, sélectifs des récepteurs $\alpha_{1a}$.
Ils peuvent donc être utilisés dans le traitement de l'incontinence urinaire.

A cet effet, ils peuvent être présentés sous toutes formes appropriées à l'administration entérale ou parentérale, en association avec des excipients pharmaceutiques, par exemple sous forme de comprimés, dragées, gélules, capsules, solutions buvables ou injectables, suppositoires, dosés pour permettre une dose journalière de 0,001 à 1000 mg de substance active.

## Annexe 1

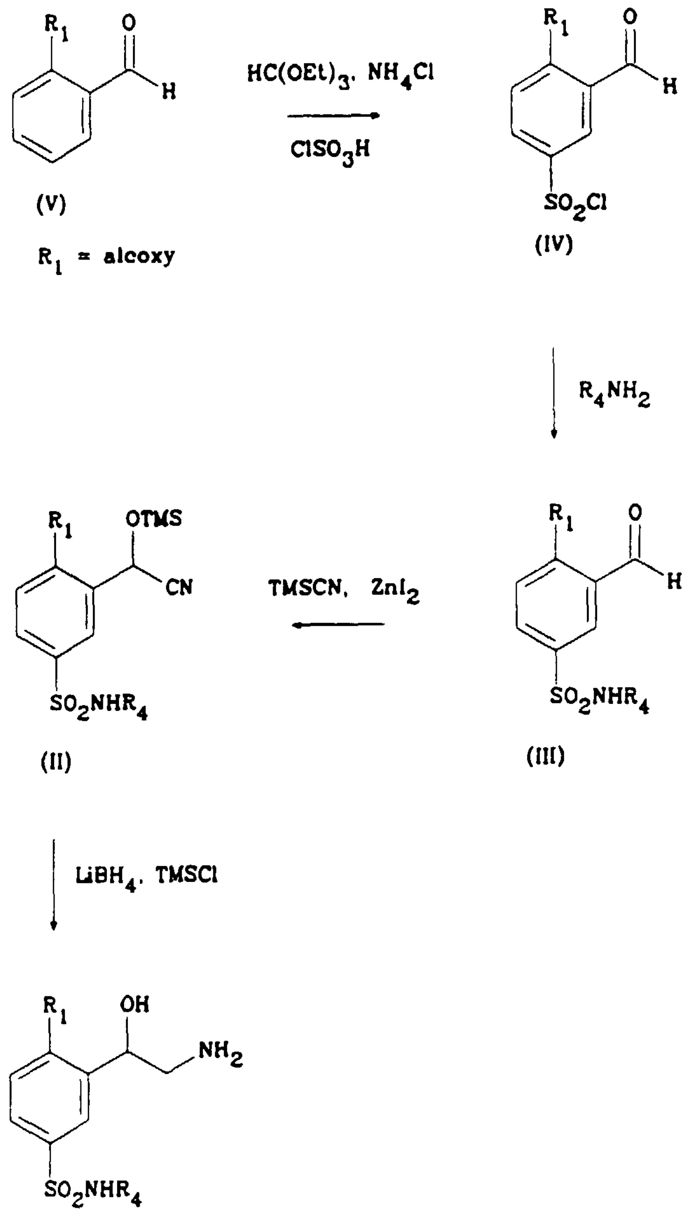


(V) $R_1$ = alcoxy

(IV)

(III)

(II)

(I) ($R_1$ = alcoxy)

## Annexe 2

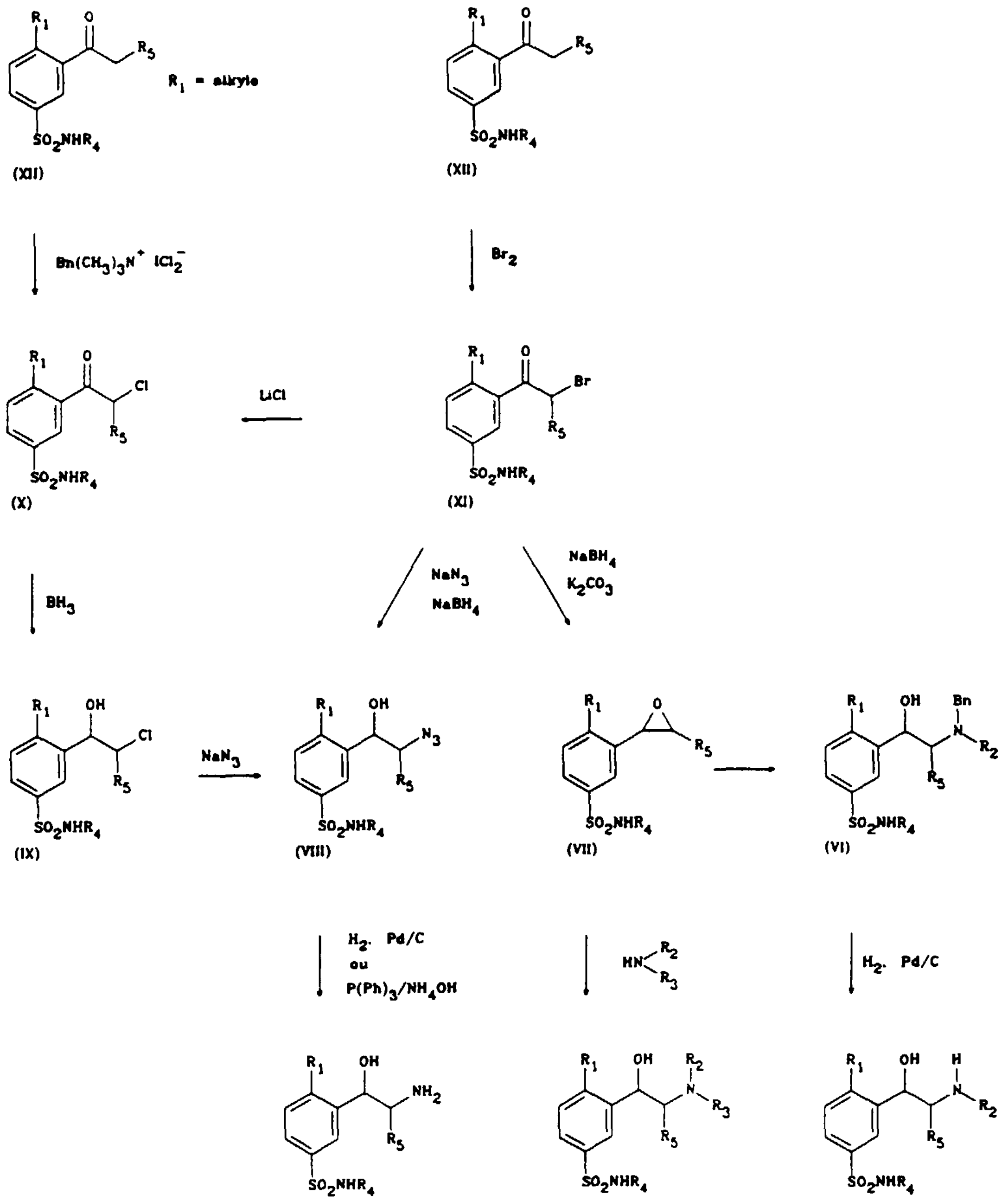


## Revendications

1. Composés de benzènesulfonamide de formule générale (I)

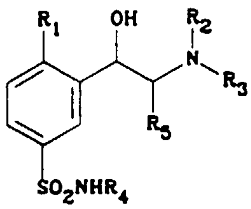

(I)

dans laquelle :

$R_1$ représente un atome d'hydrogène ou d'halogène, tel que chlore ou fluor, ou un groupe $C_{1-4}$ alkyle ou $C_{1-4}$ alcoxy, linéaire ou ramifié,
$R_2$, $R_3$, et $R_4$ représentent, indépendamment les uns des autres, des atomes d'hydrogène ou des groupes $C_{1-4}$ alkyle, linéaire, ramifié ou cyclique, et
$R_5$ représente un atome d'hydrogène, un groupe $C_{1-2}$ alkyle, $C_{1-2}$ fluoroalkyle, ou $C_{1-2}$ perfluoroalkyle,

sous forme d'énantiomères ou de diastéréoisomères, ou de mélanges de ces différentes formes, y compris de mélanges racémiques ainsi que leurs sels d'addition à des acides pharmaceutiquement acceptables.

**2.** Composés selon la revendication 1, caractérisés en ce que $R_5$ représente un atome d'hydrogène, un méthyle ou un éthyle.

**3.** Composés selon la revendication 1, caractérisés en ce que $R_1$ représente un atome d'hydrogène, un fluor, un chlore, ou un groupe $C_{1-4}$ alcoxy.

**4.** Composés selon la revendication 1, caractérisés en ce que $R_2$ et $R_3$ représentent indépendamment l'un de l'autre, un atome d'hydrogène, un méthyle, un éthyle ou un isopropyle.

**5.** Composés selon la revendication 1, caractérisés en ce que

$R_1$ représente un atome d'hydrogène, un fluor, un chlore,ou un groupe $C_{1-4}$ alcoxy,
$R_2$ et $R_3$ représentent indépendamment l'un de l'autre, un atome d'hydrogène, un méthyle, un éthyle ou un isopropyle,
$R_4$ représente, un atome d'hydrogène ou un groupe $C_{1-4}$ alkyle, linéaire, ramifié ou cyclique, et
$R_5$ représente un atome d'hydrogène, un méthyle ou un éthyle.

**6.** Composés selon la revendication 1, caractérisés en ce que

$R_1$ représente un atome d'hydrogène, un fluor, un chlore, un méthoxy ou un éthoxy,
$R_2$ et $R_3$ représentent un atome hydrogène,
$R_4$ représente, un atome d'hydrogène ou un groupe $C_{1-4}$ alkyle, linéaire, ramifié ou cyclique, et
$R_5$ représente un atome d'hydrogène ou un méthyle.

**7.** Le $\alpha$-(aminométhyl)-2-méthoxy-5-sulfonamidobenzèneméthanol, ses sels pharmaceutiquement acceptables et leurs énantiomères.

**8.** Le (+)-$\alpha$-(aminométhyl)-2-méthoxy-5-sulfonamidobenzène méthanol et ses sels pharmaceutiquement acceptables.

**9.** Le (-)-$\alpha$-(aminométhyl)-2-méthoxy-5-sulfonamidobenzène méthanol et ses sels pharmaceutiquement acceptables.

**10.** Le $\alpha$-(aminométhyl)-2-chloro-5-sulfonamidobenzèneméthanol, ses sels pharmaceutiquement acceptables et leurs énantiomères.

**11.** Le $\alpha$-(aminométhyl)-2-fluoro-5-sulfonamidobenzèneméthanol, ses sels pharmaceutiquement acceptables et leurs

énantiomères.

**12.** Le α-diéthylaminométhyl-2-méthoxy-5-sulfonamidobenzèneméthanol, ses sels pharmaceutiquement acceptables et leurs énantiomères.

**13.** Le α-méthylaminométhyl-2-méthoxy-5-sulfonamidobenzèneméthanol, ses sels pharmaceutiquement acceptables et leurs énantiomères.

**14.** Le α-aminométhyl-2-méthyl-5-sulfonamidobenzèneméthanol, ses sels pharmaceutiquement acceptables et leurs énantiomères.

**15.** Le syn ou anti (2'-méthoxy-5'-aminosulfonyl)-phenyl-2-amino-propan-1-ol, ses sels pharmaceutiquement acceptables et leurs énantiomères.

**16.** Médicament caractérisé en ce qu'il contient un composé de formule générale (I) selon la revendication 1.

**17.** Composition pharmaceutique caractérisée en ce qu'elle contient un composé de formule générale (I) selon la revendication 1, en association avec tout excipient approprié.

**18.** Procédé de préparation des composés de formule générale (I)

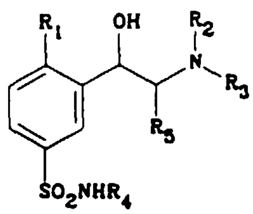

(I)

dans laquelle :

$R_1$ représente un groupe $C_{1-4}$ alcoxy, linéaire ou ramifié, et $R_2$ et $R_3$ représentent des atomes d'hydrogène,
$R_4$ représente un atomes d'hydrogène ou un groupe $C_{1-4}$ alkyle, linéaire, ramifié ou cyclique, et
$R_5$ représente un atome d'hydrogène, sous forme d'énantiomères ou de diastéréoisomères, ou de mélanges de ces différentes formes, y compris de mélanges racémiques, ainsi que leurs sels d'addition à des acides pharmaceutiquement acceptables,

qui comprend

(a) la réaction d'un composé de formule (III)

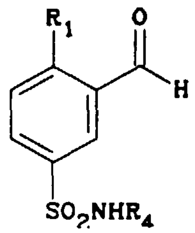

(III)

dans laquelle $R_1$ et $R_4$ sont tels que définis précédemment, avec un cyanure de triméthylsilyle, en présence d'iodure de zinc pour donner le dérivé de formule (II),

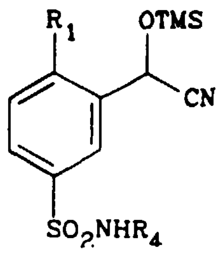

(II)

dans laquelle $R_1$ et $R_4$ sont tels que définis précédemment,
(b) la réduction du dérivé résultant (II) par le borohydrure de lithium, en présence de chlorure de trimétylsilyle, pour donner le composé résultant de formule générale (I),
(c) la transformation éventuelle de ce composé (I) résultant en ses énantiomères, diastéréoisomères, ou ses sels pharmaceutiquememnt acceptables.

**19.** Procédé de préparation des composés de formule générale (I)

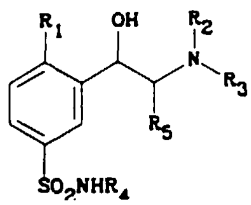

(I)

dans laquelle :

$R_1$ représente un atome d'hydrogène ou d'halogène, tel que chlore ou fluor, ou un groupe $C_{1-4}$ alkyle ou $C_{1-4}$ alcoxy, linéaire ou ramifié,
$R_2$, $R_3$, et $R_4$ représentent, indépendamment les uns des autres, des atomes d'hydrogène ou des groupes $C_{1-4}$ alkyle, linéaire, ramifié ou cyclique, et
$R_5$ représente un atome d'hydrogène, un groupe $C_{1-2}$ alkyle, $C_{1-2}$ fluoroalkyle, ou $C_{1-2}$ perfluoroalkyle, sous forme d'énantiomères ou de diastéréoisomères, ou de mélanges de ces différentes formes, y compris de mélanges racémiques, ainsi que leurs sels d'addition à des acides pharmaceutiquement acceptables,

qui comprend :

(a) soit la réaction éventuelle d'un dérivé de formule (VIII)

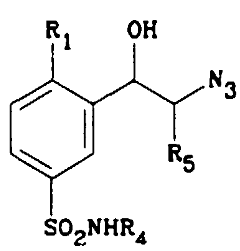

sous forme d'énantiomère ou de diastéréoisomère, ou de mélange de ces différentes formes, y compris de mélange racémiques,
dans laquelle dans laquelle $R_4$ et $R_5$ sont tels que définis précédemment avec soit de l'hydrogène en présence d'un catalyseur tel que le palladium sur charbon dans le dans où $R_1$ est défini comme précédemment à l'exception du chlore, soit avec la triphényl phosphine, puis l'ammoniaque dans le cas où $R_1$ est un atome de chlore, pour préparer les composés de formule générale (I) dans laquelle dans laquelle $R_1$, $R_4$ et $R_5$ sont tels que définis précédemment, $R_2$ et $R_3$ sont des atomes d'hydrogène,

(b) soit la réaction éventuelle d'un dérivé de formule (VII)

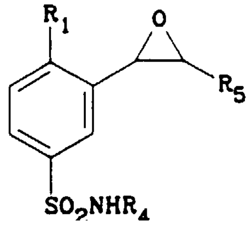

(VII)

dans laquelle dans laquelle $R_1$, $R_4$ et $R_5$ sont tels que définis précédemment, avec une amine de formule $R_2(R_3)NH$ dans laquelle $R_2$ représente un atome d'hydrogène ou un groupe $C_{1-4}$ alkyle et $R_3$ représente un groupe $C_{1-4}$ alkyle, pour obtenir le composé de formule générale (I) dans laquelle $R_2$ et $R_3$ sont tels que définis précédemment, soit par une amine de formule $R_2(Bn)NH$ dans laquelle $R_2$ est un groupe alkyle et Bn un groupe benzyle, pour obtenir un dérivé de formule (VI), qui est ensuite réduit par l'hydrogène en présence d'un catalyseur, tel que le palladium sur charbon, pour donner le composé de formule générale (I) dans la quelle $R_2$ est un groupe $C_{1-4}$ alkyle,
(c) la transformation éventuelle de ce composé (I) résultant en ses énantiomères, diastéréoisomères ou ses sels pharmaceutiquemement acceptables.

**Patentansprüche**

1. Benzolsulfonamidverbindungen der allgemeinen Formel (I)

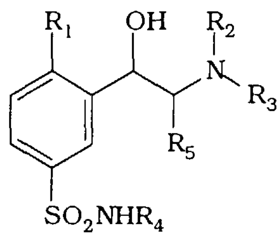

(I)

in der:

$R_1$ ein Wasserstoffatom oder ein Halogenatom, wie Chlor oder Fluor, oder eine geradkettige oder verzweigte $C_{1-4}$-Alkylgruppe oder $C_{1-4}$-Alkoxygruppe,
$R_2$, $R_3$ und $R_4$ unabhängig voneinander Wasserstoffatome oder geradkettige, verzweigte oder cyclische $C_{1-4}$-Alkylgruppen und
$R_5$ ein Wasserstoffatom, eine $C_{1-2}$-Alkylgruppe, $C_{1-2}$-Fluoralkylgruppe oder $C_{1-2}$-Perfluoralkylgruppe bedeuten,

in Form der Enantiomeren oder Diastereoisomeren oder von Mischungen dieser verschiedenen Formen, einschließlich racemischer Mischungen, sowie ihrer Additionssalze mit pharmazeutisch annehmbaren Säuren.

2. Verbindungen nach Anspruch 1, dadurch gekennzeichnet, daß $R_5$ ein Wasserstoffatom, eine Methylgruppe oder eine Ethylgruppe bedeutet.

3. Verbindungen nach Anspruch 1, dadurch gekennzeichnet, daß $R_1$ ein Wasserstoffatom, ein Fluoratom, ein Chloratom oder eine $C_{1-4}$-Alkoxygruppe darstellt.

4. Verbindungen nach Anspruch 1, dadurch gekennzeichnet, daß $R_2$ und $R_3$ unabhängig voneinander Wasserstoffatome, Methylgruppen, Ethylgruppen oder Isopropylgruppen bedeuten.

5. Verbindungen nach Anspruch 1, dadurch gekennzeichnet, daß

$R_1$ ein Wasserstoffatom, ein Fluoratom, ein Chloratom oder eine $C_{1-4}$-Alkoxygruppe,
$R_2$ und $R_3$ unabhängig voneinander Wasserstoffatome, Methylgruppen, Ethylgruppen oder Isopropylgruppen,
$R_4$ ein Wasserstoffatom oder eine geradkettige, verzweigte oder cyclische $C_{1-4}$-Alkylgruppe und
$R_5$ ein Wasserstoffatom, eine Methylgruppe oder eine Ethylgruppe bedeuten.

**6.** Verb indungen nach Anspruch 1, dadurch gekennzeichnet, daß

$R_1$ ein Wasserstoffatom, ein Fluoratom, ein Chloratom, eine Methoxygruppe oder eine Ethoxygruppe,
$R_2$ und $R_3$ ein Wasserstoffatom,
$R_4$ ein Wasserstoffatom oder eine geradkettige, verzweigte oder cyclische $C_{1-4}$-Alkylgruppe und
$R_5$ ein Wasserstoffatom oder eine Methylgruppe bedeuten.

**7.** α-(Aminomethyl)-2-methoxy-5-sulfonamidobenzolmethanol und dessen pharmazeutisch annehmbaren Salze und deren Enantiomere.

**8.** (+)-α-(Aminomethyl)-2-methoxy-5-sulfonamidobenzolmethanol und dessen pharmazeutisch annehmbaren Salze.

**9.** (-)-α-(Aminomethyl)-2-methoxy-5-sulfonamidobenzolmethanol und dessen pharmazeutisch annehmbaren Salze.

**10.** α-(Aminomethyl)-2-chlor-5-sulfonamidobenzolmethanol und dessen pharmazeutisch annehmbaren Salze und deren Enantiomere.

**11.** α-(Aminomethyl)-2-fluor-5-sulfonamidobenzolmethanol und dessen pharmazeutisch annehmbaren Salze und deren Enantiomere.

**12.** α-Diethylaminommethyl-2-methoxy-5-sulfonamidobenzolmethanol und dessen pharmazeutisch annehmbaren Salze und deren Enantiomere.

**13.** α-Methylaminomethyl-2-methoxy-5-sulfonamidobenzolmethanol und dessen pharmazeutisch annehmbaren Salze und deren Enantiomere.

**14.** α-Aminomethyl-2-methyl-5-sulfonamidobenzolmethanol und dessen pharmazeutisch annehmbaren Salze und deren Enantiomere.

**15.** syn- oder anti-(2'-Methoxy-5'-aminosulfonyl)-phenyl-2-amino-propan-1-ol, dessen pharmazeutisch annehmbaren Salze und deren Enantiomere.

**16.** Arzneimittel, dadurch gekennzeichnet, daß es eine Verbindung der allgemeinen Formel (I) nach Anspruch 1 enthält.

**17.** Pharmazeutische Zubereitung, dadurch gekennzeichnet, daß sie eine Verbindung der allgemeinen Formel (I) nach Anspruch 1 in Kombination mit irgendeinem geeigneten Trägermaterial enthält.

**18.** Verfahren zur Herstellung der Verbindungen der allgemeinen Formel (I)

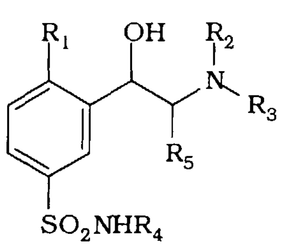

(I)

in der:

$R_1$ eine geradkettige oder verzweigte $C_{1-4}$-Alkoxygruppe,
$R_2$ und $R_3$ Wasserstoffatome,

$R_5$ ein Wasserstoffatom oder eine geradkettige, verzweigte oder cyclische $C_{1-4}$-Alkylgruppen und
$R_5$ ein Wasserstoffatom bedeuten,

in Form der Enantiomeren oder Diastereoisomeren oder von Mischungen dieser verschiedenen Formen, einschließlich racemischer Mischungen, sowie ihrer Additionssalze mit pharmazeutisch annehmbaren Säuren, welches umfaßt:

(a) die Umsetzung einer Verbindung der Formel (III)

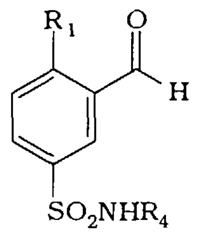
(III)

in der $R_1$ und $R_4$ die oben angegebenen Bedeutungen besitzen, in Gegenwart von Zinkiodid mit einem Trimethylsilylcyanid zur Bildung des Derivats der Formel (II)

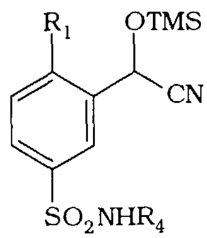
(II)

in der $R_1$ und $R_4$ die oben angegebenen Bedeutungen besitzen,
(b) die Reduktion des gebildeten Derivats (II) mit Lithiumborhydrid in Gegenwart von Trimethylsilylchlorid zur Bildung der Verbindung der allgemeinen Formel (I),
(c) die eventuelle Umwandlung der erhaltenen Verbindung der Formel (I) in ihre Enantiomeren, Diastereoisomeren oder pharmazeutisch annehmbaren Salze.

**19.** Verfahren zur Herstellung der Verbindungen der allgemeinen Formel (I)

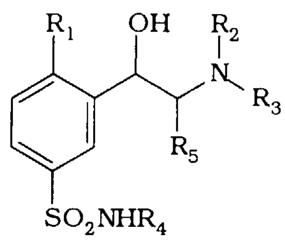
(I)

in der:

$R_1$ ein Wasserstoffatom oder ein Halogenatom, wie Chlor oder Fluor, oder eine geradkettige oder verzweigte $C_{1-4}$-Alkylgruppe oder $C_{1-4}$-Alkoxygruppe,
$R_2$, $R_3$ und $R_4$ unabhängig voneinander Wasserstoffatome oder geradkettige, verzweigte oder cyclische $C_{1-4}$-Alkylgruppen und
$R_5$ ein Wasserstoffatom, eine $C_{1-2}$-Alkylgruppe, $C_{1-2}$-Fluoralkylgruppe oder $C_{1-2}$-Perfluoralkylgruppe bedeuten,

in Form der Enantiomeren oder Diastereoisomeren oder von Mischungen dieser verschiedenen Formen, einschließlich racemischer Mischungen, sowie ihrer Additionssalze mit pharmazeutisch annehmbaren Säuren,

welches umfaßt:

(a) entweder die eventuelle Reaktion eines Derivats der Formel (VIII)

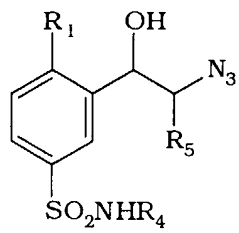

(VIII)

in Form des Enantiomeren oder Diastereoisomeren oder einer Mischung dieser verschiedenen Formen einschließlich racemischer Mischungen,
worin $R_4$ und $R_5$ die oben angegebenen Bedeutungen besitzen, mit entweder Wasserstoff in Gegenwart eines Katalysators, wie Palladium-auf-Kohlenstoff, in dem Fall, wo $R_1$ die oben angegebenen Bedeutungen mit Ausnahme von Chlor besitzt, oder mit Triphenylphosphin und dann mit Ammoniak in dem Fall, da $R_1$ ein Chloratom darstellt, zur Herstellung der Verbindungen der allgemeinen Formel (I), in der $R_1$, $R_4$ und $R_5$ die oben angegebenen Bedeutungen besitzen und $R_2$ und $R_3$ Wasserstoffatome bedeuten,
(b) oder die eventuelle Umsetzung eines Derivats der Formel (VII)

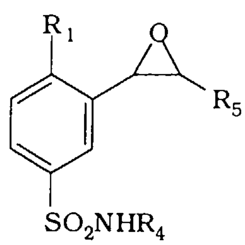

(VII)

in der $R_1$, $R_4$ und $R_5$ die oben angegebenen Bedeutungen besitzen, mit einem Amin der Formel $R_2(R_3)NH$, worin $R_2$ ein Wasserstoffatom oder eine $C_{1-4}$-Alkylgruppe und $R_3$ eine $C_{1-4}$-Alkylgruppe bedeuten, zur Bildung der Verbindung der allgemeinen Formel (I), in der $R_2$ und $R_3$ die oben angegebenen Bedeutungen besitzen, oder mit einem Amin der Formel $R_2(Bn)NH$, worin $R_2$ eine Alkylgruppe und Bn eine Benzylgruppe bedeuten, zur Bildung eines Derivats der Formel (VI), welches anschließend mit Wasserstoff in Gegenwart eines Katalysators, wie Palladium-auf-Kohlenstoff, reduziet wird zur Bildung der Verbindung der allgemeinen Formel (I), in der $R_2$ eine $C_{1-4}$-Alkylgruppe darstellt,
(c) die eventuelle Umwandlung dieser erhaltenen Verbindung (I) in ihre Enantiomeren, Diastereoisomeren oder pharmazeutisch annehmbaren Salze.

## Claims

**1.** Benzenesulphonamide compounds of general formula (I)

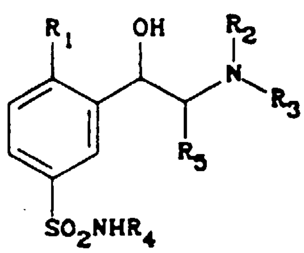

(I)

in which:

$R_1$ represents a hydrogen atom, a halogen atom such as chlorine or fluorine or a linear or branched $C_{1-4}$ alkyl or $C_{1-4}$ alkoxy group,
$R_2$, $R_3$ and $R_4$ represent, independently of one another, hydrogen atoms or linear, branched or cyclic $C_{1-4}$ alkyl groups, and
$R_5$ represents a hydrogen atom or a $C_{1-2}$ alkyl, $C_{1-2}$ fluoroalkyl or $C_{1-2}$ perfluoroalkyl group,

in the form of enantiomers or diastereoisomers or mixtures of these different forms, including racemic mixtures, as well as their addition salts with pharmaceutically acceptable acids.

**2.** Compounds according to Claim 1, characterized in that $R_5$ represents a hydrogen atom, a methyl or an ethyl.

**3.** Compounds according to Claim 1, characterized in that $R_1$ represents a hydrogen atom, a fluorine, a chlorine or a $C_{1-4}$ alkoxy group.

**4.** Compounds according to Claim 1, characterized in that $R_2$ and $R_3$ represent, independently of one another, a hydrogen atom, a methyl, an ethyl or an isopropyl.

**5.** Compounds according to Claim 1, characterized in that

$R_1$ represents a hydrogen atom, a fluorine, a chlorine or a $C_{1-4}$ alkoxy group,
$R_2$ and $R_3$ represent, independently of one another, a hydrogen atom, a methyl, an ethyl or an isopropyl,
$R_4$ represents a hydrogen atom or a linear, branched or cyclic $C_{1-4}$ alkyl group, and
$R_5$ represents a hydrogen atom, a methyl or an ethyl.

**6.** Compounds according to Claim 1, characterized in that

$R_1$ represents a hydrogen atom, a fluorine, a chlorine, a methoxy or an ethoxy,
$R_2$ and $R_3$ represent a hydrogen atom,
$R_4$ represents a hydrogen atom or a linear, branched or cyclic $C_{1-4}$ alkyl group, and
$R_5$ represents a hydrogen atom or a methyl.

**7.** α-(Aminomethyl)-2-methoxy-5-sulphonamidobenzenemethanol, its pharmaceutically acceptable salts and their enantiomers.

**8.** (+)-α-(Aminomethyl)-2-methoxy-5-sulphonamidobenzenemethanol and its pharmaceutically acceptable salts.

**9.** (-)-α-(Aminomethyl)-2-methoxy-5-sulphonamidobenzenemethanol and its pharmaceutically acceptable salts.

**10.** α-(Aminomethyl)-2-chloro-5-sulphonamidobenzenemethanol, its pharmaceutically acceptable salts and their enantiomers.

**11.** α-(Aminomethyl)-2-fluoro-5-sulphonamidobenzenemethanol, its pharmaceutically acceptable salts and their enantiomers.

**12.** α-Diethylaminomethyl-2-methoxy-5-sulphonamidobenzenemethanol, its pharmeceutically acceptable salts and their enantiomers.

**13.** α-Methylaminomethyl-2-methoxy-5-sulphonamidobenzenemethanol, its pharmeceutically acceptable salts and their enantiomers.

**14.** α-Aminomethyl-2-methyl-5-sulphonamidobenzenemethanol, its pharmeceutically acceptable salts and their enantiomers.

**15.** Syn or anti (2'-methoxy-5'-aminosulphonyl)-phenyl-2-aminopropan-1-ol, its pharmaceutically acceptable salts and their enantiomers.

**16.** Medicinal product, characterized in that it contains a compound of general formula (I) according to Claim 1.

**17.** Pharmaceutical composition, characterized in that it contains a compound of general formula (I) according to Claim 1, in combination with any suitable excipient.

**18.** Process for preparing the compounds of general formula (I)

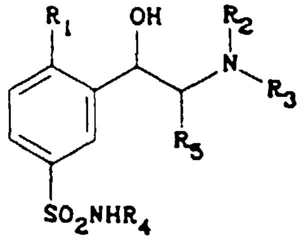

(I)

in which:

$R_1$ represents a linear or branched $C_{1-4}$ alkoxy group, and
$R_2$ and $R_3$ represent hydrogen atoms,
$R_4$ represents a hydrogen atom or a linear, branched or cyclic $C_{1-4}$ alkyl group, and
$R_5$ represents a hydrogen atom, in the form of enantiomers or diastereoisomers or mixtures of these different forms, including racemic mixtures, as well as their addition salts with pharmaceutically acceptable acids,

which comprises:

(a) the reaction of a compound of formula (III)

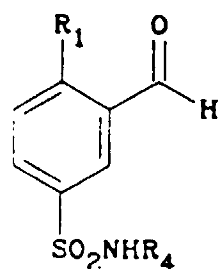

(III)

in which $R_1$ and $R_4$ are as defined above, with a trimethylsilyl cyanide in the presence of zinc iodide, to give the derivative of formula (II)

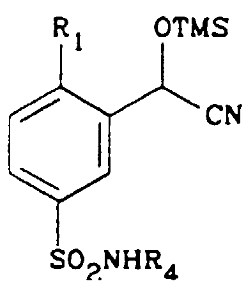

(II)

in which $R_1$ and $R_4$ are as defined above,
(b) the reduction of the resulting derivative (II) with lithium borohydride in the presence of trimethylsilyl chloride, to give the resulting compound of general formula (I),
(c) the optional conversion of this resulting compound (I) to its enantiomers or diastereoisomers or its pharmaceutically acceptable salts.

**19.** Process for preparing the compounds of general formula (I)

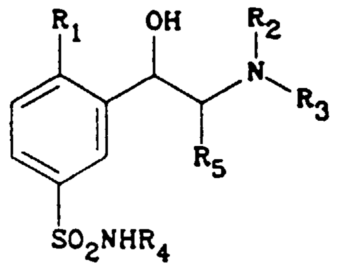

(I)

in which:

$R_1$ represents a hydrogen atom, a halogen atom such as chlorine or fluorine or a linear or branched $C_{1-4}$ alkyl or $C_{1-4}$ alkoxy group,
$R_2$, $R_3$ and $R_4$ represent, independently of one another, hydrogen atoms or linear, branched or cyclic $C_{1-4}$ alkyl groups, and
$R_5$ represents a hydrogen atom or a $C_{1-2}$ alkyl, $C_{1-2}$ fluoroalkyl or $C_{1-2}$ perfluoroalkyl group, in the form of enantiomers or diastereoisomers or mixtures of these different forms, including racemic mixtures, as well as their addition salts with pharmaceutically acceptable acids,

which comprises:

(a) either the possible reaction of a derivative of formula (VIII)

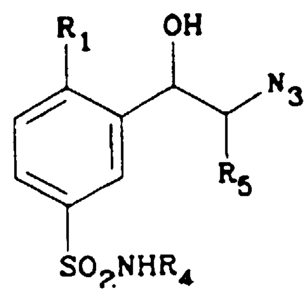

in the form of an enantiomer or diastereoisomer or mixture of these different forms, including a racemic mixture, in which $R_4$ and $R_5$ are as defined above, either with hydrogen in the presence of a catalyst such as palladium on charcoal in the case where $R_1$ is defined as above with the exception of chlorine, or with triphenylphosphine and then aqueous ammonia in the case where $R_1$ is a chlorine atom, to prepare the compounds of general formula (I) in which $R_1$, $R_4$ and $R_5$ are as defined above and $R_2$ and $R_3$ are hydrogen atoms,
(b) or the possible reaction of a derivative of formula (VII)

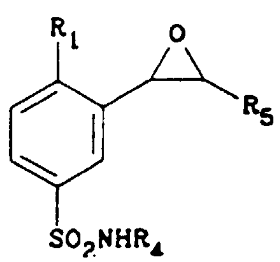

(VII)

in which $R_1$, $R_4$ and $R_5$ are as defined above, with an amine of formula $R_2(R_3)NH$ in which $R_2$ represents a

hydrogen atom or a $C_{1-4}$ alkyl group and $R_3$ represents a $C_{1-4}$ alkyl group, to obtain the compound of general formula (I) in which $R_2$ and $R_3$ are as defined above, or with an amine of formula $R_2(Bn)NH$ in which $R_2$ is an alkyl group and Bn a benzyl group, to obtain a derivative of formula (VI), which is thereafter reduced with hydrogen in the presence of a catalyst such as palladium on charcoal, to give the compound of general formula (I) in which $R_2$ is a $C_{1-4}$ alkyl group,
(c) the optional conversion of this resulting compound (I) to its enantiomers or diastereoisomers or its pharmaceutically acceptable salts.